(19) Europäisches Patentamt
European Patent Office
Office européen des brevets

(11) EP 1 625 114 B1

(12) **EUROPEAN PATENT SPECIFICATION**

(45) Date of publication and mention of the grant of the patent:
**27.08.2008 Bulletin 2008/35**

(21) Application number: **04731523.9**

(22) Date of filing: **06.05.2004**

(51) Int Cl.:
***C07D 211/44*** (2006.01) ***C07D 211/26*** (2006.01)
***A61K 31/445*** (2006.01)

(86) International application number:
**PCT/SE2004/000693**

(87) International publication number:
**WO 2004/099144 (18.11.2004 Gazette 2004/47)**

(54) **Piperidine derivatives for the the treatment of chemokine or H1 mediated diseases**

PIPERIDIN-DERIVATEN UND DEREN VERWENDUNG BEI DER BEHANDLUNG VON DURCH CHEMOKINEN ODER H1 VERMITTELTEN KRANKHEITEN

PIPERIDINE DERIVES POUR LE TRAITEMENT DE MALADIES FAISANT INTERVENIR CHIMIOKINES OU H1

(84) Designated Contracting States:
**AT BE BG CH CY CZ DE DK EE ES FI FR GB GR HU IE IT LI LU MC NL PL PT RO SE SI SK TR**

(30) Priority: **09.05.2003 SE 0301368**

(43) Date of publication of application:
**15.02.2006 Bulletin 2006/07**

(73) Proprietor: **AstraZeneca AB**
**151 85 Södertälje (SE)**

(72) Inventors:
- **LUCKHURST, Christopher**
  **Leicestershire LE11 5RH (GB)**
- **MOCHEL, Tobias**
  **Leicestershire LE11 5RH (GB)**
- **PERRY, Matthew**
  **Leicestershire LE11 5RH (GB)**
- **SPRINGTHORPE, Brian**
  **Leicestershire LE11 5RH (GB)**
- **STEIN, Linda**
  **Leicestershire LE11 5RH (GB)**

(56) References cited:
- **No relevant documents have been disclosed.**

EP 1 625 114 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

## Description

The present invention concerns piperidine derivatives having pharmaceutical activity, to processes for preparing such derivatives, to pharmaceutical compositions comprising such derivatives and to the use of such derivatives as active therapeutic agents.

Pharmaceutically active piperidine derivatives are disclosed in WO99/38514, WO99/04794 and WO00/35877. Piperidine derivatives having chemokine antagonism activity are disclosed in WO 01/77101. WP 02/081449 and WO 02/079194.

Histamine is a basic amine, 2-(4-imidazolyl)-ethylamine, and is formed from histidine by histidine decarboxylase. It is found in most tissues of the body, but is present in high concentrations in the lung, skin and in the gastrointestinal tract. At the cellular level inflammatory cells such as mast cells and basophils store large amounts of histamine. It is recogniscd that the degranulation of mast cells and basophils and the subsequent release of histamine is a fundamental mechanism responsible for the clinical manifestation of an allergic process. Histamine produces its actions by an effect on specific histamine G-protein coupled receptors, which are of three main types, H1, H2 and H3. Histamine H1 antagonists comprise the largest class of medications used in the treatment of patients with allergic disorders, especially rhinitis and urticaria. H1 antagonists are useful in controlling the allergic response by for example blocking the action of histamine on post-capillary venule smooth muscle, resulting in decreased vascular permeability, exudation and oedema. The antagonists also produce blockade of the actions of histamine on the H1 receptors on c-type nociceptive nerve fibres, resulting in decreased itching and sneezing.

Chemokines are chemotactic cytokines that are released by a wide variety of cells to attract macrophages, T cells, eosinophils, basophils and neutrophils to sites of inflammation and also play a rôle in the maturation of cells of the immune system. Chemokines play an important rôle in immune and inflammatory responses in various diseases and disorders, including asthma and allergic diseases, as well as autoimmune pathologies such as rheumatoid arthritis and atherosclerosis. These small secreted molecules are a growing superfamily of 8-14 kDa proteins characterised by a conserved four cysteine motif. The chemokine superfamily can be divided into two main groups exhibiting characteristic structural motifs, the Cys-X-Cys (C-X-C, or $\alpha$) and Cys-Cys (C-C, or $\beta$) families. These are distinguished on the basis of a single amino acid insertion between the NH-proximal pair of cysteine residues and sequence similarity.

The C-X-C chemokines include several potent chemoattractants and activators of neutrophils such as interleukin-8 (IL-8) and neutrophil-activating peptide 2 (NAP-2).

The C-C chemokines include potent chemoattractants of monocytes and lymphocytes but not neutrophils such as human monocyte chemotactic proteins 1-3 (MCP-1, MCP-2 and MCP-3), RANTES (Regulated on Activation, Normal T Expressed and Secreted), eotaxin and the macrophage inflammatory proteins 1$\alpha$ and 1$\beta$ (MIP-1$\alpha$ and MIP-1$\beta$).

Studies have demonstrated that the actions of the chemokines are mediated by subfamilies of G protein-coupled receptors, among which are the receptors designated CCR1, CCR2, CCR2A, CCR2B, CCR3, CCR4, CCR5, CCR6, CCR7, CCR8, CCR9, CCR10, CXCR1, CXCR2, CXCR3 and CXCR4. These receptors represent good targets for drug development since agents which modulate these receptors would be useful in the treatment of disorders and diseases such as those mentioned above.

Viral infections are known to cause lung inflammation. It has been shown experimentally that the common cold increases mucosal output of eotaxin in the airways. Instillation of eotaxin into the nose can mimic some of the signs and symptoms of a common cold. (See, Greiff L et al Allergy (1999) 54(11) 1204-8 [Experimental common cold increase mucosal output of eotaxin in atopic individuals] and Kawaguchi M et al Int. Arch. Allergy Immunol. (2000) 122 S1 44 [Expression of eotaxin by normal airway epithelial cells after virus A infection].)

The present invention provides a compound of formula (I):

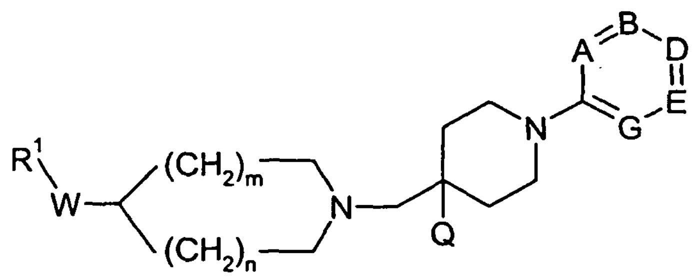

(I)

wherein:

n and m are, independently, 0 or 1;

A, B, D, E, G represent one each of $CCO_2R^5$, $CR^2$, $CR^3$, $CR^4$, and CH or N,
Q is hydrogen or hydroxy;
W is $CH_2$, O, NH or N($C_{1-4}$ alkyl);
$R^1$ is phenyl optionally substituted by halogen, cyano, $C(O)NH_2$, $C_{1-4}$ alkyl, $C_{1-4}$ haloalkyl, $C_{1-4}$ alkoxy or $C_{1-4}$ haloalkoxy;
$R^2$, $R^3$ and $R^4$ are, independently, hydrogen, halogen, cyano, nitro, hydroxy, $NR^6R^7$, $C_{1-6}$ alkyl (optionally substituted with halogen), $C_{1-6}$ alkoxy (optionally substituted with halogen), $S(O)_p$($C_{1-6}$ alkyl), $S(O)_qCF_3$ or $S(O)_2NR^7R^8$;
$R^5$ is hydrogen, $C_{1-6}$ alkyl or benzyl;
p and q are, independently, 0, 1 or 2;
$R^6$, $R^7$, $R^8$ and $R^9$ are, independently, hydrogen, $C_{1-6}$ alkyl (optionally substituted by halogen, hydroxy or $C_{3-6}$ cycloalkyl), $CH_2$($C_{2-5}$ alkenyl), phenyl (itself optionally substituted by halogen, hydroxy, nitro, $NH_2$, NH($C_{1-4}$ alkyl), N($C_{1-4}$ alkyl)$_2$ (and these alkyl groups may join to form a ring as described for $R^6$ and $R^7$ below), $S(O)_2$($C_{1-4}$ alkyl), $S(O)_2NH_2$, $S(O)_2NH$($C_{1-4}$ alkyl), $S(O)_2N$($C_{1-4}$ alkyl)$_2$ (and these alkyl groups may join to form a ring as described for $R^6$ and $R^7$ below), cyano, $C_{1-4}$ alkyl, $C_{1-4}$ alkoxy, $C(O)NH_2$, C(O)NH($C_{1-4}$ alkyl), C(O)N($C_{1-4}$ alkyl)$_2$ (and these alkyl groups may join to form a ring as described for $R^6$ and $R^7$ below), $CO_2H$, $CO_2$($C_{1-4}$ alkyl), NHC(O)($C_{1-4}$ alkyl), $NHS(O)_2$($C_{1-4}$ alkyl), C(O)($C_{1-4}$ alkyl), $CF_3$ or $OCF_3$) or heterocyclyl (itself optionally substituted by halogen, hydroxy, nitro, $NH_2$, NH($C_{1-4}$ alkyl), N($C_{1-4}$ alkyl)$_2$ (and these alkyl groups may join to form a ring as described for $R^6$ and $R^7$ below), $S(O)_2$($C_{1-4}$ alkyl), $S(O)_2NH_2$, $S(O)_2NH$($C_{1-4}$ alkyl), $S(O)_2N$($C_{1-4}$ alkyl)$_2$ (and these alkyl groups may join to form a ring as described for $R^6$ and $R^7$ below), cyano, $C_{1-4}$ alkyl, $C_{1-4}$ alkoxy, $C(O)NH_2$, C(O)NH($C_{1-4}$ alkyl), C(O)N($C_{1-4}$ alkyl)$_2$ (and these alkyl groups may join to form a ring as described for $R^6$ and $R^7$ below), $CO_2H$, $CO_2$($C_{1-4}$ alkyl), NHC(O)($C_{1-4}$ alkyl), $NHS(O)_2$($C_{1-4}$ alkyl), C(O)($C_{1-4}$ alkyl), $CF_3$ or $OCF_3$);
alternatively $NR^6R^7$ or $NR^8R^9$ may, independently, form a 4-7 membered heterocyclic ring, azetidine, pyrrolidine, piperidine, azepine, morpholine or piperazine, the latter optionally substituted by $C_{1-4}$ alkyl on the distal nitrogen;
or an N-oxide thereof; or a pharmaceutically acceptable salt thereof; or a solvate thereof.

Certain compounds of the present invention can exist in different isomeric forms (such as enantiomers, diastereomers, geometric isomers or tautomers). The present invention covers all such isomers and mixtures thereof in all proportions.

Suitable salts include acid addition salts such as a hydrochloride, dihydrochloride, hydrobromide, phosphate, sulfate, acetate, diacetate, fumarate, maleate, tartrate, citrate, oxalate, methanesulfonate or p-toluenesulfonate.

The compounds of the invention may exist as solvates (such as hydrates) and the present invention covers all such solvates.

Halogen includes fluorine, chlorine, bromine and iodine. Halogen is, for example, fluorine or chlorine.

Alkyl groups and moieties are straight or branched chain and comprise, for example, 1 to 6 (such as 1 to 4) carbon atoms. Examples of alkyl groups are methyl, ethyl, n-propyl, iso-propyl or tert-butyl.

Haloalkyl groups and moieties comprise an alkyl part, as defined above, and one or more (for example 1 to 6) of the same or different halogen atoms. Haloalkyl is, for example, $CF_3$.

Alkenyl groups comprise, for example, 2 to 6 (such as 2 to 4) carbon atoms. Examples of alkenyl groups are vinyl or allyl.

In one embodiment cycloalkyl groups comprise from 3 to 6 carbon atoms and are monocyclic. Cycloalkyl is, for example, cyclopropyl, cyclopentyl or cyclohexyl.

Heterocyclyl is an aromatic or non-aromatic 5 or 6 membered ring, optionally fused to one or more other rings, comprising at least one heteroatom selected from the group comprising nitrogen, oxygen and sulfur; or an N-oxide thereof, or an S-oxide or S-dioxide thereof. Heterocyclyl is, for example, furyl, thienyl (also known as thiophenyl), pyrrolyl, 2,5-dihydropyrrolyl, thiazolyl, pyrazolyl, oxazolyl, isoxazolyl, imidazolyl, piperidinyl, morpholinyl, pyridinyl, dihydropyridinyl (for example in a 6-oxo-1,6-dihydro-pyridinyl moiety), pyrimidinyl, indolyl, 2,3-dihydroindolyl, benzo[b]furyl (also known as benzfuryl), benz[b]thienyl (also known as benzthienyl or benzthiophenyl), 2,3-dihydrobenz[b]thienyl (for example in a 1-dioxo-2,3-dihydrobenz[b]thienyl moiety), indazolyl, benzimidazolyl, benztriazolyl, benzoxazolyl, benzthiazolyl (for example in a 1H-benzthiazol-2-one-yl moiety), 2,3-dihydrobenzthiazolyl (for example in a 2,3-dihydrobenzthiazol-2-one-yl moiety), 1,2,3-benzothiadiazolyl, an imidazopyridinyl (such as imidazo[1,2a]pyridinyl), thieno[3,2-b]pyridin-6-yl, 1,2,3-benzoxadiazolyl, benzo[1,2,3]thiadiazolyl, 2,1,3-benzothiadiazolyl, benzofurazan (also known as 2,1,3-benzoxadiazolyl), quinoxalinyl, dihydro-1-benzopyryliumyl (for example in a coumarinyl or a chromonyl moiety), 3,4-dihydro-1H-2,1-benzothiazinyl (for example in a 2-dioxo-3,4-dihydro-1H-2,1-benzothiazinyl moiety), a pyrazolopyridine (for example 1H-pyrazolo[3,4-b]pyridinyl), a purine (for example in a 3,7-dihydro-purin-2,6-dione-8-yl moiety), quinolinyl, isoquinolinyl, dihydroisoquinolinyl (for example in a 2H-isoquinolin-1-one-yl moiety), a naphthyridinyl (for example [1,6] naphthyridinyl or [1,8]naphthyridinyl), a dihydro[1,8]naphthyridinyl (for example in a 1H-[1,8]naphthyridin-4-one-yl moiety), a benzothiazinyl, a dihydrobenzothiazinyl (for example in a 4H-benzo[1,4]thiazin-3-one-yl moiety), benzo[d]imidazo [2,1-b]thiazol-2-yl or dibenzothiophenyl (also known as dibenzothienyl); or an N-oxide thereof, or an S-oxide or S-dioxide

thereof.

An N-oxide of a compound of formula (I) is, for example, a 1-oxy-[1,4']bipiperidinyl-1'-yl compound.

In one particular aspect the invention provides a compound of formula (I)

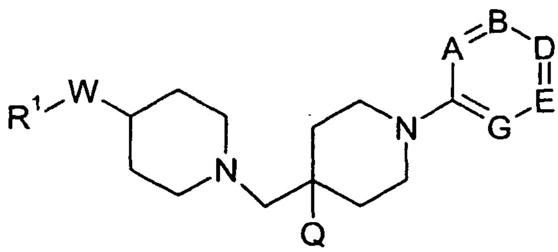

(I)

wherein:

n and m are both 1;
A, B, D, E, G represent one each of $CCO_2R^5$, $CR^2$, $CR^3$, $CR^4$, and CH or N;
Q is hydrogen or hydroxy;
W is $CH_2$, O, NH or N($C_{1-4}$ alkyl);
$R^1$ is phenyl optionally substituted by halogen, cyano, $C_{1-4}$ alkyl, $C_{1-4}$ haloalkyl, $C_{1-4}$ alkoxy or $C_{1-4}$ haloalkoxy;
$R^2$, $R^3$ and $R^4$ are, independently, hydrogen, halogen, cyano, nitro, hydroxy, $NR^6R^7$, $C_{1-6}$ alkyl (optionally substituted with halogen), $C_{1-6}$ alkoxy (optionally substituted with halogen), $S(O)_p$($C_{1-6}$ alkyl), $S(O)_qCF_3$ or $S(O)_2NR^7R^8$;
$R^5$ is hydrogen, $C_{1-6}$ alkyl or benzyl;
p and q are, independently, 0, 1 or 2;
$R^6$, $R^7$, $R^8$ and $R^9$ are, independently, hydrogen, $C_{1-6}$ alkyl (optionally substituted by halogen, hydroxy or $C_{3-6}$ cycloalkyl), $CH_2$($C_{2-5}$ alkenyl), phenyl (itself optionally substituted by halogen, hydroxy, nitro, $NH_2$, NH($C_{1-4}$ alkyl), N($C_{1-4}$ alkyl)$_2$ (and these alkyl groups may join to form a ring as described for $R^6$ and $R^7$ below), $S(O)_2$($C_{1-4}$ alkyl), $S(O)_2NH_2$, $S(O)_2NH$($C_{1-4}$ alkyl), $S(O)_2N$($C_{1-4}$ alkyl)$_2$ (and these alkyl groups may join to form a ring as described for $R^6$ and $R^7$ below), cyano, $C_{1-4}$ alkyl, $C_{1-4}$ alkoxy, C(O)$NH_2$, C(O)NH($C_{1-4}$ alkyl), C(O)N($C_{1-4}$ alkyl)$_2$ (and these alkyl groups may join to form a ring as described for $R^6$ and $R^7$ below), $CO_2H$, $CO_2$($C_{1-4}$ alkyl), NHC(O)($C_{1-4}$ alkyl), $NHS(O)_2$($C_{1-4}$ alkyl), C(O)($C_{1-4}$ alkyl), $CF_3$ or $OCF_3$) or heterocyclyl (itself optionally substituted by halogen, hydroxy, nitro, $NH_2$, NH($C_{1-4}$ alkyl), N($C_{1-4}$ alkyl)$_2$ (and these alkyl groups may join to form a ring as described for $R^6$ and $R^7$ below), $S(O)_2$($C_{1-4}$ alkyl), $S(O)_2NH_2$, $S(O)_2NH$($C_{1-4}$ alkyl), $S(O)_2N$($C_{1-4}$ alkyl)$_2$ (and these alkyl groups may join to form a ring as described for $R^6$ and $R^7$ below), cyano, $C_{1-4}$ alkyl, $C_{1-4}$ alkoxy, C(O)$NH_2$, C(O)NH($C_{1-4}$ alkyl), C(O)N($C_{1-4}$ alkyl)$_2$ (and these alkyl groups may join to form a ring as described for $R^6$ and $R^7$ below), $CO_2H$, $CO_2$($C_{1-4}$ alkyl), NHC(O)($C_{1-4}$ alkyl), $NHS(O)_2$($C_{1-4}$ alkyl), C(O)($C_{1-4}$ alkyl), $CF_3$ or $OCF_3$);
alternatively $NR^6R^7$ or $NR^8R^9$ may, independently, form a 4-7 membered heterocyclic ring, azetidine, pyrrolidine, piperidine, azepine, morpholine or piperazine, the latter optionally substituted by $C_{1-4}$ alkyl on the distal nitrogen;
or an N-oxide thereof; or a pharmaceutically acceptable salt thereof; or a solvate thereof.

In a further aspect the present invention provides a compound wherein W is O.

In a still further aspect the present invention provides a compound of formula (I) where n and m are both 1.

In another aspect $R^1$ is phenyl optionally substituted (for example independently mono- or di-substituted) with halogen (for example chlorine or fluorine), cyano, $C_{1-4}$ alkyl (for example methyl) or $C_{1-4}$ alkoxy (for example methoxy).

In yet another aspect $R^1$ is phenyl optionally substituted (for example independently mono- or di-substituted) with halogen (for example chlorine or fluorine), $C_{1-4}$ alkyl (for example methyl) or $C_{1-4}$ alkoxy (for example methoxy).

In a further aspect $R^1$ is phenyl optionally substituted (for example with one, two or three of the same or different) with fluorine, chlorine, cyano, $C_{1-4}$ alkyl (for example methyl) or $C_{1-4}$ alkoxy (for example methoxy). In a still further aspect $R^1$ is phenyl substituted by one, two or three (for example two or three) substituents independently selected from: fluorine, chlorine, cyano and methyl. For example $R^1$ is 3,4-dichlorophenyl, 2,4-dichloro-3-methylphenyl, 3,4-dichloro-2-methylphenyl, 2,4-dichlorophenyl, 4-chloro-2-methylphenyl, 2-chloro-4-fluorophenyl, 4-fluorophenyl or 3-chloro-4-cyanophenyl.

In a still further aspect of the invention Q is hydrogen.

In yet another aspect of the invention $R^5$ is hydrogen.

In a further aspect of the invention A is $CCO_2R^5$, for example wherein $R^5$ is hydrogen or $C_{1-4}$ alkyl (such as methyl).

In a still further aspect of the invention one of A, B, D, E, G represent one each of $CCO_2R^5$, $CR^2$, $CR^3$, $CR^4$, and CH or N; wherein $R^2$, $R^3$ and $R^4$, are, independently, hydrogen, halogen, cyano, nitro, $C_{1-4}$ alkyl (such as methyl or ethyl), $C_{1-4}$ alkoxy (such as methoxy or ethoxy), $CF_3$, $OCF_3$, $S(O)_2(C_{1-4}$ alkyl) (such as $S(O)_2CH_3$) or $S(O)_2NH_2$; and $R^5$ is hydrogen or $C_{1-6}$ alkyl (such as methyl, ethyl or *tert*-butyl).

In yet another aspect of the invention one of B, D, E and G is CH and the others are $CR^2$, $CR^3$ and $CR^4$, wherein $R^2$, $R^3$ and $R^4$, are, independently, hydrogen, halogen, cyano, $C_{1-4}$ alkyl (such as methyl or ethyl), $C_{1-4}$ alkoxy (such as methoxy or ethoxy), $CF_3$, $OCF_3$, $S(O)_2(C_{1-4}$ alkyl) (such as $S(O)_2CH_3$) or $S(O)_2NH_2$ {for example $R^2$, $R^3$ and $R^4$, are, independently, hydrogen, halogen, cyano, nitro, $C_{1-4}$ alkyl (such as methoxy or ethoxy), or $C_{1-4}$ alkoxy (such as methoxy or ethoxy), $CF_3$ or $OCF_3$}. In a further aspect of the invention B, D, E and G are all CH.

In a further aspect of the invention A, B, D, E, G represent one each of $CCO_2R^5$, $CR^2$, $CR^3$, $CR^4$, and CH or N; wherein $R^2$, $R^3$ and $R^4$ are, independently, hydrogen, halogen (such as fluorine or chlorine), $C_{1-4}$ alkyl (such as methyl), $C_{1-4}$ alkoxy (such as methoxy), nitro or $CF_3$; and $R^5$ is hydrogen or $C_{1-6}$ alkyl (such as methyl, ethyl or *tert*-butyl).

In another aspect the present invention provides a compound of formula (I) wherein: Q is hydrogen; W is O or $CH_2$; A is $CCO_2R^5$; B, D, E and G are all CH; $R^1$ is phenyl substituted by halogen (for example by one or two chlorine atoms), cyano or $C_{1-4}$ alkyl (for example methyl); and $R^5$ is hydrogen or $C_{1-4}$ alkyl (for example methyl).

In a further aspect the present invention provides a compound of formula (I) wherein n and m are, independently, 0 or 1, $R^1$ is phenyl optionally substituted by halogen (such as fluorine or chlorine), cyano, $C_{1-4}$ alkyl (such as methyl) or $C(O)NH_2$; W is O or $CH_2$; Q is H or OH; A, B, D, E, G represent one each of $CCO_2R^5$, $CR^2$, $CR^3$, $CR^4$, and CH or N; wherein $R^2$, $R^3$ and $R^4$ are, independently, hydrogen, halogen (such as fluorine or chlorine), $C_{1-4}$ alkyl (such as methyl), $C_{1-4}$ alkoxy (such as methoxy), nitro or $CF_3$; and $R^5$ is hydrogen or $C_{1-6}$ alkyl (such as methyl, ethyl or *tert*-butyl).

The compounds of the present invention can be prepared as described below.

A compound of formula (I) can be prepared by nucleophilic substitution of fluoride (II):

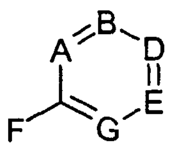 (II)

with a compound of formula (III):

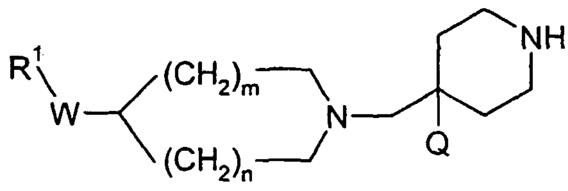 (III)

in the presence of a suitable solvent (such as N,N-dimethylformamide), at a suitable temperature (for example in the range 10-100°C, such as 70-90°C), optionally in the presence of a suitable base (such as potassium carbonate).

For a compound of formula (I):

- when $R^5$ is hydrogen said compound may be converted to a compound of the invention where $R^5$ is not hydrogen by a standard esterification method well known in the art; and,
- when $R^5$ is not hydrogen said compound may be converted to a compound of the invention where $R^5$ is hydrogen by a standard ester hydrolysis method well known in the art.

A compound of formula (III) can be prepared by deprotecting a compound of formula (IV):

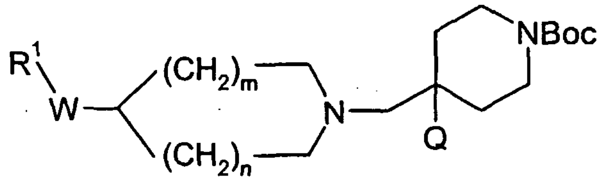 (IV)

for example using trifluoroacetic acid in a suitable solvent (such as dichloromethane) or using a source of hydrogen chloride in a suitable solvent (such as dioxane).

A compound of formula (IV), wherein Q is hydrogen, can be prepared by reacting a compound of formula (V):

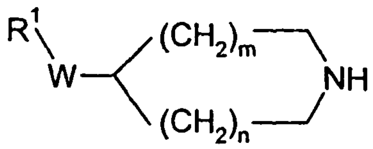 (V)

with a compound of formula (VI):

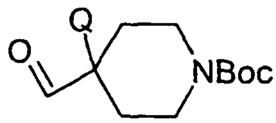 (VI)

in the presence of $NaBH(OAc)_3$ and acetic acid, in a suitable solvent (such as tetrahydrofuran or dichloromethane).

A compound of formula (IV), wherein Q is hydroxy, can be prepared by reacting a compound of formula (V) with a compound of formula (VII):

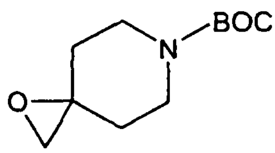 (VII)

in a suitable solvent (such as a $C_{1-6}$ aliphatic alcohol, for example ethanol) at room temperature.

Alternatively, a compound of formula (I) can be prepared by a Buchwald-Hartwig amination, where a compound of formula (VIII):

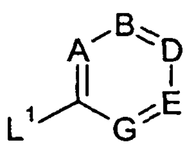 (VIII)

wherein $L^1$ is bromo or iodo; is reacted with a compound of formula (III) in the presence of a suitable palladium compound (such as tris-(dibenzylidineacetone)dipalladium), a ligand (such as 2-dicyclohexylphosphino-2'-(N,N-dimethylamino)biphenyl), a base (such as caesium carbonate) and a solvent (such as a solvent comprising a ether, for example dioxin) at a suitable temperature (such as in the range 80-100°C).

The preparation of various intermediates can be found in WO00/66559 and WO01/77101; alternatively they can be prepared by using or adapting literature methods.

Further compounds of formula (I) can be prepared by adaptation of: the routes described above, methods described in the art or the Examples recited below.

Compounds of formula (II) to (VII) can be prepared by using or adapting methods described in the art. The preparation of various phenoxy piperidines is described in WO 01/77101.

In the above processes it may be desirable or necessary to protect an acid group or a hydroxy or other potentially reactive group. Suitable protecting groups and details of processes for adding and removing such groups may be found in "Protective Groups in Organic Synthesis", 3rd Edition (1999) by Greene and Wuts.

In another aspect the present invention provides processes for the preparation of compounds of formula (I).

The compounds of formula (I) have activity as pharmaceuticals, in particular as modulators of chemokine receptor (especially CCR3) activity, and may be used in the treatment of autoimmune, inflammatory, proliferative or hyperproliferative diseases, or immunologically-mediated diseases (including rejection of transplanted organs or tissues and Acquired Immunodeficiency Syndrome (AIDS)).

Examples of these conditions are:

(1) (the respiratory tract) obstructive diseases of airways including: chronic obstructive pulmonary disease (COPD) (such as irreversible COPD); asthma {such as bronchial, allergic, intrinsic, extrinsic or dust asthma, particularly chronic or inveterate asthma (for example late asthma or airways hyper-responsiveness)}; bronchitis {such as eosinophilic bronchitis}; acute, allergic, atrophic rhinitis or chronic rhinitis including rhinitis caseosa, hypertrophic rhinitis, rhinitis purulenta, rhinitis sicca or rhinitis medicamentosa; membranous rhinitis including croupous, fibrinous or pseudomembranous rhinitis or scrofulous rhinitis; seasonal rhinitis including rhinitis nervosa (hay fever) or vasomotor rhinitis; sarcoidosis; farmer's lung and related diseases; nasal polyposis; fibroid lung, idiopathic interstitial pneumonia, antitussive activity, treatment of chronic cough associated with inflammatory conditions of the airways or iatrogenic induced cough;

(2) (bone and joints) arthrides including rheumatic, infectious, autoimmune, seronegative spondyloarthropathies (such as ankylosing spondylitis, psoriatic arthritis or Reiter's disease), Behçet's disease, Sjogren's syndrome or systemic sclerosis;

(3) (skin and eyes) psoriasis, atopic dermatitis, contact dermatitis or other eczmatous dermitides, seborrhoetic dermatitis, lichen planus, phemphigus, bullous phemphigus, epidermolysis bullosa, urticaria, angiodermas, vasculitides erythemas, cutaneous eosinophilias, uveitis, alopecia areata, corneal ulcer or vernal conjunctivitis;

(4) (gastrointestinal tract) Coeliac disease, proctitis, eosinophilic gastro-enteritis, mastocytosis, Crohn's disease, ulcerative colitis, irritable bowel disease or food-related allergies which have effects remote from the gut (for example migraine, rhinitis or eczema);

(5) (Allograft rejection) acute and chronic following, for example, transplantation of kidney, heart, liver, lung, bone marrow, skin or cornea; or chronic graft versus host disease; and/or

(6) (other tissues or diseases) Alzheimer's disease, multiple sclerosis, atherosclerosis, Acquired Immunodeficiency Syndrome (AIDS), lupus disorders (such as lupus erythematosus or systemic lupus), erythematosus, Hashimoto's thyroiditis, myasthenia gravis, type I diabetes, nephrotic syndrome, eosinophilia fascitis, hyper IgE syndrome, leprosy (such as lepromatous leprosy), peridontal disease, Sezary syndrome, idiopathic thrombocytopenia pupura or disorders of the menstrual cycle.

The compounds of formula (I) or a pharmaceutically acceptable salt thereof or a solvate thereof, are also H1 antagonists (and can, therefore, be used in the treatment of allergic disorders); and may also be used to control a sign and/or symptom of what is commonly referred to as a cold (for example a sign and/or symptom of a common cold or influenza or other associated respiratory virus infection).

The invention also provides a compound of the formula (I), or a pharmaceutically acceptable salt thereof or a solvate thereof, for use in therapy.

In another aspect the invention provides the use of a compound of formula (I), or a pharmaceutically acceptable salt thereof or a solvate thereof, in the manufacture of a medicament for use in therapy (for example modulating chemokine receptor activity (especially CCR3 receptor activity), antagonising H1 or treating a sign and/or symptom of what is commonly referred to as a cold).

The invention further provides the use of a compound of formula (I), or a pharmaceutically acceptable salt thereof, in the manufacture of a medicament for use in the treatment of:

(1) (the respiratory tract) obstructive diseases of airways including: chronic obstructive pulmonary disease (COPD) (such as irreversible COPD); asthma {such as bronchial, allergic, intrinsic, extrinsic or dust asthma, particularly chronic or inveterate asthma (for example late asthma or airways hyper-responsiveness)}; bronchitis {such as eosinophilic bronchitis}; acute, allergic, atrophic rhinitis or chronic rhinitis including rhinitis caseosa, hypertrophic rhinitis, rhinitis purulenta, rhinitis sicca or rhinitis medicamentosa; membranous rhinitis including croupous, fibrinous or pseudomembranous rhinitis or scrofulous rhinitis; seasonal rhinitis including rhinitis nervosa (hay fever) or vasomotor rhinitis; sarcoidosis; farmer's lung and related diseases; nasal polyposis; fibroid lung, idiopathic interstitial

pneumonia, antitussive activity, treatment of chronic cough associated with inflammatory conditions of the airways or iatrogenic induced cough;

(2) (bone and joints) arthrides including rheumatic, infectious, autoimmune, seronegative spondyloarthropathies (such as ankylosing spondylitis, psoriatic arthritis or Reiter's disease), Behçet's disease, Sjogren's syndrome or systemic sclerosis;

(3) (skin and eyes) psoriasis, atopic dermatitis, contact dermatitis or other eczmatous dermitides, seborrhoetic dermatitis, lichen planus, phemphigus, bullous phemphigus, epidermolysis bullosa, urticaria, angiodermas, vasculitides erythemas, cutaneous eosinophilias, uveitis, alopecia areata, corneal ulcer or vernal conjunctivitis;

(4) (gastrointestinal tract) Coeliac disease, proctitis, eosinophilic gastro-enteritis, mastocytosis, Crohn's disease, ulcerative colitis, irritable bowel disease or food-related allergies which have effects remote from the gut (for example migraine, rhinitis or eczema);

(5) (Allograft rejection) acute and chronic following, for example, transplantation of kidney, heart, liver, lung, bone marrow, skin or cornea; or chronic graft versus host disease; and/or

(6) (other tissues or diseases) Alzheimer's disease, multiple sclerosis, atherosclerosis, Acquired Immunodeficiency Syndrome (AIDS), lupus disorders (such as lupus erythematosus or systemic lupus), erythematosus, Hashimoto's thyroiditis, myasthenia gavis, type I diabetes, nephrotic syndrome, eosinophilia fascitis, hyper IgE syndrome, leprosy (such as lepromatous leprosy), Peridontal disease, sezary syndrome, idiopathic thrombocytopenia pupura or disorders of the menstrual cycle;

in a mammal (for example man).

In a still further aspect a compound of formula (I), or a pharmaceutically acceptable salt thereof, is useful in the treatment of asthma.

The present invention also provides the use of a compound of formula (I), or a pharmaceutically acceptable salt thereof, in the manufacture of a medicament for use in the treatment of asthma {such as bronchial, allergic, intrinsic, extrinsic or dust asthma, particularly chronic or inveterate asthma (for example late asthma or airways hyper-responsiveness)}; or rhinitis {including acute, allergic, atrophic or chronic rhinitis, such as rhinitis caseosa, hypertrophic rhinitis, rhinitis purulenta, rhinitis sicca or rhinitis medicamentosa; membranous rhinitis including croupous, fibrinous or pseudomembranous rhinitis or scrofulous rhinitis; seasonal rhinitis including rhinitis nervosa (hay fever) or vasomotor rhinitis}.

In order to use a compound of the invention, or a pharmaceutically acceptable salt thereof or solvate thereof, for the therapeutic treatment of a mammal, such as man, said ingredient is normally formulated in accordance with standard pharmaceutical practice as a pharmaceutical composition. Therefore in another aspect the present invention provides a pharmaceutical composition which comprises a compound of the formula (I), or a pharmaceutically acceptable salt thereof or a solvate thereof (active ingredient), and a pharmaceutically acceptable adjuvant, diluent or carrier.

In a further aspect the present invention provides a process for the preparation of said composition which comprises mixing active ingredient with a pharmaceutically acceptable adjuvant, diluent or carrier. Depending on the mode of administration, the pharmaceutical composition will, for example, comprise from 0.05 to 99 %w (per cent by weight), such as from 0.05 to 80 %w, for example from 0.10 to 70 %w, such as from 0.10 to 50 %w, of active ingredient, all percentages by weight being based on total composition.

The pharmaceutical compositions of this invention may be administered in standard manner for the disease condition that it is desired to treat, for example by topical (such as to the lung and/or airways or to the skin), oral, rectal or parenteral administration. For these purposes the compounds of this invention may be formulated by means known in the art. A suitable pharmaceutical composition of this invention is one suitable for oral administration in unit dosage form, for example a tablet or capsule which contains between 0.1mg and 1g of active ingredient.

Each patient may receive, for example, a dose of $0.01mgkg^{-1}$ to $100mgkg^{-1}$, such as in the range of $0.1mgkg^{-1}$ to $20mgkg^{-1}$, of the active ingredient administered, for example, 1 to 4 times per day.

While not forming part of the invention, compounds of the invention can be used in combination therapies wherein a compound of formula (1) or a pharmaceutically acceptable salt, solvate or *in vivo* hydrolysable ester thereof, or a pharmaceutical composition or formulation comprising a compound of formula (1) is administered concurrently or sequentially or as a combined preparation with another therapeutic agent or agents, for the treatment of one or more of the conditions listed.

A compound of the invention can be used combined with agents such as:- Non-steroidal anti-inflammatory agents (hereinafter NSAIDs) including non-selective cyclo-oxygenase (COX)-1 / COX-2 inhibitors whether applied topically or systemically (such as piroxicam, diclofenac, propionic acids such as naproxen, flurbiprofen, fenoprofen, ketoprofen and ibuprofen, fenamates such as mefenamic acid, indomethacin, sulindac, azapropazone, pyrazolones such as phenylbutazone, salicylates such as aspirin); selective COX-2 inhibitors (such as meloxicam, celecoxib, rofecoxib, valdecoxib, lumarocoxib, parecoxib and etoricoxib); cyclo-oxygenase inhibiting nitric oxide donors (CINODs); glucocorticosteroids (whether administered by topical, oral, intramuscular, intravenous, or intra-articular routes); methotrexate, leflunomide; hydroxychloroquine, d-penicillamine, auranofin or other parenteral or oral gold preparations ; analgesics;

diacerein; intra-articular therapies such as hyaluronic acid derivatives; and nutritional supplements such as glucosamine, for use in the treatment of the inflammatory diseases such as (but not restricted to) rheumatoid arthritis, osteoarthritis, asthma, allergic rhinitis, chronic obstructive pulmonary disease (COPD), psoriasis, and inflammatory bowel disease.

Described is also the combination of a compound of the invention together with a cytokine or agonist or antagonist of cytokine function, (including agents which act on cytokine signalling pathways such as modulators of the SOCS system) including alpha-, beta-, and gamma-interferons; insulin-like growth factor type I (IGF-1); interleukins (IL) including IL1 to 17, and interleukin antagonists or inhibitors such as anakinra; tumour necrosis factor alpha (TNF-$\alpha$) inhibitors such as anti-TNF monoclonal antibodies (for example infliximab; adalimumab , and CDP-870) and TNF receptor antagonists including immunoglobulin molecules (such as etanercept) and low-molecular-weight agents such as pentoxyfylline.

Further described is the combination of a compound of the invention together with modulators of chemokine receptor function such as antagonists of CCR1, CCR2, CCR2A, CCR2B, CCR4, CCR5, CCR6, CCR7, CCR8, CCR9, CCR10 and CCR11 (for the C-C family); CXCR1, CXCR2, CXCR3, CXCR4 and CXCR5 (for the C-X-C family) and $CX_3CR1$ for the C-$X_3$-C family.

Further described is the combination of a compound of the invention together with an inhibitor of matrix metalloproteases (MMPs), i.e., the stromelysins, the collagenases, and the gelatinases, as well as aggrecanase; especially collagenase-1 (MMP-1), callagenase-2 (MMP-8), collagenase-3 (MMP-13), stromelysin-1 (MMP-3), stromelysin-2 (MMP-10), and stromelysin-3 (MMP-11) and MMP-9 and MMP-12, including agents such as doxycycline.

Further described is the combination of a compound of the invention together with a leukotriene biosynthesis inhibitor, 5-lipoxygenase (5-LO) inhibitor or 5-lipoxygenase activating protein (FLAP) antagonist such as; zileuton; ABT-761; fenleuton; tepoxalin; Abbott-79175; Abbott-85761; N-(5-substituted)-thiophenc-2-alkylsulfonamides; 2,6-di-tert-butylphenolhydrazones; methoxytetrahydropyrans such as Zeneca ZD-2138; the compound SB-210661; pyridinyl-substituted 2-cyanonaphthalene compounds such as L-739,010; 2-cyanoquinoline compounds such as L-746,530; indole and quinoline compounds such as MK-591, MK-886, and BAY x 1005.

Further described is the combination of a compound of the invention together with a receptor antagonist for leukotrienes (LT) B4, LTC4, LTD4, and LTE4. selected from the group consisting of the phenothiazin-3-1s such as L-651,392; amidino compounds such as CGS-25019c; benzoxalamines such as ontazolast; benzenecarboximidamides such as BIIL 284/260; and compounds such as zafirlukast, ablukast, montelukast, pranlukast, verlukast (MK-679), RG-12525, Ro-245913, iralukast (CGP 45715A), and BAY x 7195.

Further described is the combination of a compound of the invention together with a phosphodiesterase (PDE) inhibitor such as the methylxanthines including theophylline and aminophylline; and selective PDE isoenzyme inhibitors including PDE4 inhibitors and inhibitors of the isoform PDE4D, and inhibitors of PDE5.

Further described is the combination of a compound of the invention together with histamine type 1 receptor antagonists such as cetirizine, loratadine, desloratadine, fexofenadine, acrivastine, terfenadine, astemizole, azelastine, levocabastine, chlorpheniramine, promethazine, cyclizine, and mizolastine applied orally, topically or parenterally.

Further described is the combination of a compound of the invention together with a proton pump inhibitor (such as omeprazole) or gastroprotective histamine type 2 receptor antagonist.

Further described is the combination of a compound of the invention with antagonists of the histamine type 4 receptor.

Further described is the combination of a compound of the invention together with an alpha-1/alpha-2 adrenoceptor agonist vasoconstrictor sympathomimetic agent, such as propylhexedrine, phenylephrine, phenylpropanolamine, ephedrine, pseudoephedrine, naphazoline hydrochloride, oxymetazoline hydrochloride, tetrahydrozoline hydrochloride, xylometazoline hydrochloride, tramazoline hydrochloride, and ethylnorepinephrine hydrochloride.

Further described is the combination of a compound of the invention together with anticholinergic agents including muscarinic receptor (M1, M2, and M3) antagonists such as atropine, hyoscine, glycopyrrrolate, ipratropium bromide, tiotropium bromide, oxitropium bromide, pirenzepine, and telenzepine.

Further described is the combination of a compound of the invention together with a beta-adrenoceptor agonist (including beta receptor subtypes 1-4) such as isoprenaline, salbutamol, formoterol, salmeterol, terbutaline, orciprenaline, bitolterol mesylate, and pirbuterol, including chiral enantiomers thereof.

Further described is the combination of a compound of the invention together with a chromone, including sodium cromoglycate and nedocromil sodium.

Further described is the combination of a compound of the invention together with a glucocorticoid, such as flunisolide, triamcinolone acetonide, beclomethasone dipropionate, budesonide, fluticasone propionate, ciclesonide, and mometasone furoate.

Further described is the combination of a compound of the invention together with an agent that modulate nuclear hormone receptors such as PPARs.

Further described is the combination of a compound of the invention together with an immunoglobulin (Ig) or Ig preparation or an antagonist or antibody modulating Ig function such as anti-IgE (e.g. omalizumab).

Further described is the combination of a compound of the invention together with other systemic or topically-applied anti-inflammatory agents including thalidomide and derivatives, retinoids, dithranol, and calcipotriol.

Further described is the combination of a compound of the invention together with combinations of aminosalicylates and sulfapyridine such as sulfasalazine, mesalazine, balsalazide, and olsalazine; and immunomodulatory agents such as the thiopurines, and corticosteroids such as budesonide.

Further described is the combination of a compound of the invention together with an antibacterial agent including penicillin derivatives, tetracyclines, macrolides, beta-lactams, fluoroquinolones, metronidazole, and inhaled aminoglycosides; and antiviral agents including acyclovir, famciclovir, valaciclovir, ganciclovir, cidofovir; amantadine, rimantadine; ribavirin; zanamavir and oseltamavir; protease inhibitors such as indinavir, nelfinavir, ritonavir, and saquinavir; nucleoside reverse transcriptase inhibitors such as didanosine, lamivudine, stavudine, zalcitabine, zidovudine; non-nucleoside reverse transcriptase inhibitors such as nevirapine, efavirenz.

Further described is the combination of a compound of the invention together with cardiovascular agents such as calcium channel blockers, beta-adrenoceptor blockers, angiotensin-converting enzyme (ACE) inhibitors, angiotensin-2 receptor antagonists; lipid lowering agents such as statins, and fibrates; modulators of blood cell morphology such as pentoxyfylline; thrombolytics, and anticoagulants including platelet aggregation inhibitors.

Further described is the combination of a compound of the invention together with CNS agents such as antidepressants (such as sertraline), anti-Parkinsonian drugs (such as deprenyl, L-dopa, ropinirole, pramipexole, MAOB inhibitors such as selegine and rasagiline, comP inhibitors such as tasmar, A-2 inhibitors, dopamine reuptake inhibitors, NMDA antagonists, nicotine agonists, dopamine agonists and inhibitors of neuronal nitric oxide synthase), and anti-Alzheimer's drugs such as donepezil, rivastigmine, tacrine, COX-2 inhibitors, propentofylline or metrifonate.

Further described is the combination of a compound of the invention together with agents for the treatment of acute and chronic pain, including centrally and peripherally-acting analgesics such as opioid analogues and derivatives, carbamazepine, phenytoin, sodium valproate, amitryptiline and other antidepressant agents, paracetamol, and non-steroidal anti-inflammatory agents.

Further described is the combination of a compound of the invention together with parenterally or topically-applied (including inhaled) local anaesthetic agents such as lignocaine and analogues.

The compounds of the present invention may also be used in combination with anti-osteoporosis agents including hormonal agents such as raloxifene, and biphosphonates such as alendronate.

Further described is the combination of a compound of the invention together with (i) tryptase inhibitors; (ii) platelet activating factor (PAF) antagonists; (iii) interleukin converting enzyme (ICE) inhibitors; (iv) IMPDH inhibitors; (v) adhesion molecule inhibitors including VLA-4 antagonists; (vi) cathepsins; (vii) Kinase inhibitors including but not limited to inhibitors of tyrosine kinases (such as Btk, Itk, Jak3 MAP examples of inhibitors might include Gefitinib, Imatinib mesylate), Serine / threonine kinases (including but not limited to inhibitors of MAP kinases such as p38, JNK, protein kinases A, B and C and IKK), and kinases involved in cell cycle regulation (such as but not limted to the cylin dependent kinases); (viii) glucose-6 phosphate dehydrogenase inhibitors; (ix) kinin-$B_1$ - and $B_2$ -receptor antagonists; (x) anti-gout agents, e.g., colchicine; (xi) xanthine oxidase inhibitors, e.g., allopurinol; (xii) uricosuric agents, e.g., probenecid, sulfinpyrazone, and benzbromarone; (xiii) growth hormone secretagogues; (xiv) transforming growth factor (TGF$\beta$); (xv) platelet-derived growth factor (PDGF); (xvi) fibroblast growth factor, e.g., basic fibroblast growth factor (bFGF); (xvii) granulocyte macrophage colony stimulating factor (GM-CSF); (xviii) capsaicin cream; (xix) tachykinin $NK_1$ and $NK_3$ receptor antagonists such as the group consisting of NKP-608C; SB-233412 (talnetant); and D-4418; (xx) elastase inhibitors such as the group consisting of UT-77 and ZD-0892; (xxi) TNF-alpha converting enzyme inhibitors (TACE); (xxii) induced nitric oxide synthase (iNOS) inhibitors or (xxiii) chemoattractant receptor-homologous molecule expressed on TH2 cells, (such as CRTH2 antagonists) (xxiv) inhibitors of P38 (xxv) agents modulating the function of Toll-like receptors (TLR) and (xxvi) agents modulating the activity of purinergic receptors such as P2X7; (xxvii) inhibitors of transcription factors activation such as NFkB, API, and STATS.

The compounds of the invention can also be used in combination with existing therapeutic agents for the treatment of cancer. Suitable agents to be used in combination include:

(i) antiproliferative/antineoplastic drugs and combinations thereof, as used in medical oncology, such as alkylating agents (for example cis-platin, carboplatin, cyclophosphamide, nitrogen mustard, melphalan, chlorambucil, busulphan and nitrosoureas); antimetabolites (for example antifolates such as fluoropyrimidines like 5-fluorouracil and tegafur, raltitrexed, methotrexate, cytosine arabinoside, hydroxyurea, gemcitabine and paclitaxel; antitumour antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere); and topoisomerase inhibitors (for example epipodophyllotoxins like etoposide and teniposide, amsacrine, topotecan and camptothecins);

(ii) cytostatic agents such as antioestrogens (for example tamoxifen, toremifene, raloxifene, droloxifene and iodoxyfene), oestrogen receptor down regulators (for example fulvestrant), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5α-reductase such as finasteride;
(iii) Agents which inhibit cancer cell invasion (for example metalloproteinase inhibitors like marimastat and inhibitors of urokinase plasminogen activator receptor function);
(iv) inhibitors of growth factor function, for example such inhibitors include growth factor antibodies, growth factor receptor antibodies (for example the anti-erbb2 antibody trastuzumab and the anti-erbb1 antibody cetuximab [C225]), farnesyl transferase inhibitors, tyrosine kinase inhibitors and serine/threonine kinase inhibitors, for example inhibitors of the epidermal growth factor family (for example EGFR family tyrosine kinase inhibitors such as N-(3-chloro-4-fluorophenyl)-7-methoxy-6-(3-morpholinopropoxy)quinazolin-4-amine (gefitinib, AZD 1839), N-(3-ethynylphenyl)-6,7-bis(2-methoxyethoxy)quinazolin-4-amine (erlotinib, OSI-774) and 6-acrylamido-N-(3-chloro-4-fluorophenyl)-7-(3-morpholinopropoxy)quinazolin-4-amine (CI 1033)), for example inhibitors of the platelet-derived growth factor family and for example inhibitors of the hepatocyte growth factor family;
(v) antiangiogenic agents such as those which inhibit the effects of vascular endothelial growth factor, (for example the anti-vascular endothelial cell growth factor antibody bevacizumab, compounds such as those disclosed in International Patent Applications WO 97/22596, WO 97/30035, WO 97/32856 and WO 98/13354) and compounds that work by other mechanisms (for example linomide, inhibitors of integrin αvβ3 function and angiostatin);
(vi) vascular damaging agents such as combretastatin A4 and compounds disclosed in International Patent Applications WO 99/02166, WO 00/40529, WO 00/41669, WO 01/92224, WO 02/04434 and WO 02/08213;
(vii) antisense therapies, for example those which are directed to the targets listed above, such as ISIS 2503, an anti-ras antisense;
(viii) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; and
(ix) immunotherapeutic approaches, including for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

The invention will now be illustrated by the following examples in which, unless stated otherwise:
(i) when given, $^1$H NMR data is quoted and is in the form of delta values for major diagnostic protons, given in parts per million (ppm) relative to tetramethylsilane (TMS) as an internal standard, determined at 300MHz or 400MHz using perdeuterio DMSO-D6 ($CD_3SOCD_3$) or $CDCl_3$ as the solvent unless otherwise stated;
(ii) mass spectra (MS) were run with an electron energy of 70 electron volts in the chemical ionisation (CI) mode using a direct exposure probe; where indicated ionisation was effected by electron impact (EI) or fast atom bombardment (FAB); where values for m/z are given, generally only ions which indicate the parent mass are reported, and unless otherwise stated the mass ion quoted is the positive mass ion - $(M+H)^+$;
(iii) the title and sub-title compounds of the examples and methods were named using the name program from Advanced Chemistry Development Inc version 6.0;
(iv) unless stated otherwise, reverse phase HPLC was conducted using a Symmetry™, NovaPak™ or Xerra™ reverse phase silica column; and
(v) the following abbreviations are used:

| | | | |
|---|---|---|---|
| RPHPLC | Reverse phase HPLC | DMSO | Dimethylsulfoxide |
| HPLC | high pressure liquid chromatography | aq | Aqueous |
| TFA | Trifluoroacetic acid | RT | room temperature |
| TMEDA | Tetramethylethylenediamine | THF | Tetrahydrofuran |

Intermediate I

This illustrates the preparation of 4-(3,4-dichlorophenoxy)-1-(piperidin-4-ylmethyl)piperidine

a) *tert*-butyl 4-{[4-(3,4-dichlorophenoxy)piperidin-1-yl]methyl}piperidine-1-carboxylate

4-(3,4-Dichlorophenoxy)piperidine (1.27 g) was dissolved in tetrahydrofuran (20 mL); acetic acid (0.5 mL) and *tert*-butyl 4-formylpiperidine-1-carboxylate (1.43 g) were added to the solution. The reaction mixture was stirred at room temperature for 30 min then sodium triacetoxyborohydride (1.53 g) was added and the mixture was stirred at room temperature overnight. The reaction mixture was poured into 2M sodium hydroxide solution (50 mL) and product was extracted with diethyl ether. The combined ether extracts were washed with brine, dried, filtered and evaporated. Crude material was purified by flash chromatography, (eluting with 979:20:1 dichloromethane : methanol: aqueous ammonia) to give the sub-title compound (2.15 g).
MS 443/445 $[M+H]^+$ (ES+)
$^1H$ NMR δ ($CDCl_3$) 1.06 (2H, ddd), 1.45 (9H, s), 1.61 - 1.82 (5H, m), 1.92 - 1.98 (2H, m), 2.16 - 2.27 (4H, m), 2.65 - 2.73 (4H, m), 4.08 (2H, d), 4.25 (1H, dq), 6.75 (1H, dd), 6.99 (1H, d), 7.30 (1H, d)

b) 4-(3,4-Dichlorophenoxy)-1-(piperidin-4-ylmethyl)-piperidine

*tert*-butyl 4-{[4-(3,4-dichlorophenoxy)piperidin-1-yl]methyl}piperidine-1-carboxylate (1.0 g) was added to a mixture of 20% TFA in dichloromethane (20 mL) and the mixture was stirred at room temperature for 1 h. Solvent was removed by evaporation and 2M sodium hydroxide solution (25 mL) was added to the residue. The product was extracted with ethyl acetate and the organic phase was washed with brine, dried, filtered and evaporated to give the title compound (0.5 g).
MS 343/345 $[M+H]^+$ (ES+)
$^1H$ NMR δ ($CDCl_3$) 1.10 (2H, qd), 1.60 (1H, qquintet), 1.73 - 1.83 (4H, m), 1.90 - 2.01 (2H, m), 2.16 - 2.26 (4H, m), 2.55 - 2.70 (4H, m), 3.09 (2H, d), 4.24 (1H, dquintet), 6.75 (1H, dd), 6.99 (1H, d), 7.27 (1H, d)

The following Intermediates were prepared analogously from the appropriate aryloxy piperidine or arylmethylpiperidine:

| Intermediate | Name (M+H) | $^1H$ NMR δ ($CDCl_3$) |
|---|---|---|
| 2 | 4-(2,4-Dichloro-3-methylphenoxy)-1-(piperidin-4-ylmethyl)-piperidine (357/359) | 1.13 - 1.27 (2H, m), 1.57 - 1.70 (1H, m), 1.76 - 2.00 (2H, m), 2.16 - 2.32 (4H, m), 2.46 (3H, s), 2.60 - 2.99 (8H, m), 3.16 (2H, d), 4.31 (1H, quintet), 6.75 (1H, d), 7.18 (1H, d) |
| 3 | 4-(4-Chloro-2-methylphenoxy)-1-(piperidin-4-ylmethyl)-piperidine (322/324) | 1.08 - 1.21 (2H, m), 1.56 - 1.68 (1H, m), 1.73 - 1.86 (4H, m), 1.90 - 1.99 (2H, m), 2.16 - 2.31 (7H, m), 2.57 - 2.69 (4H, m), 3.12 (2H, d), 4.23 - 4.31 (1H, m), 6.74 (1H, d), 7.06 (1H, dd), 7.11 (1H, d) |
| 4 | 4-(3,4-Dichloro-2-methylphenoxy)-1-(piperidin-4-ylmethyl)-piperidine (357/359) | ($CD_3OD$) 1.10 - 1.22 (2H, m), 1.66 - 1.85 (5H, m), 1.94 - 2.04 (2H, m), 2.22 (2H, d), 2.31 (3H, s), 2.32 - 2.41 (2H, m), 2.59 - 2.72 (4H, m), 3.08 (2H, d), 4.38 - 4.46 (1H, m), 6.91 (1H, d), 7.27 (1H, d) |
| 5 | 2-Chloro-4-[[1-(piperidin-4-ylmethyl)-4-piperidinyl]oxy]-benzonitrile (333/335) | ($CD_3OD$) 1.21 - 1.32 (2H, m), 1.74 - 1.90 (5H, m), 1.99 - 2.10 (2H, m), 2.26 (2H, d), 2.31 - 2.40 (2H, m), 2.67 - 2.79 (4H, m), 3.11 - 3.21 (2H, m), 4.52 - 4.62 (1H, m), 7.05 (1H, dd), 7.21 (1H, d), 7.70 (1H, d) |
| 6 | 4-[(4-Fluorophenyl)methyl]-1-(piperidin-4-ylmethyl)-piperidine ((291) Prepared from 4-fluorobenzylpiperidine (*Bioorganic & medicinal chemistry letters*, **2000,** *10*, 1377) | ($CD_3OD$+DMSO) 1.19 - 1.32 (4H, m), 1.46 - 1.54 (1H, m), 1.55 - 1.62 (2H, m), 1.77 - 1.84 (1H, m), 1.85 - 1.93 (4H, m), 2.17 (2H, d), 2.51 (2H, d); 2.80 - 2.89 (4H, m), 3.23 - 3.26 (2H, m), 7.01 (2H, t), 7.16 (2H, dd) |
| 7 | 4-(4-Chloro-3-methylphenoxy)-1-(piperidin-4-ylmethyl)piperidine (322/324) | 1.07 - 1.21 (2H, m), 1.63 - 1.80 (5H, m), 1.94 - 2.01 (2H, m), 2.21 (2H, d), 2.25 - 2.34 (5H, m), 2.58 (2H, td), 2.66 - 2.75 (2H, m), 2.99 - 3.06 (2H, m), 4.30 - 4.38 (1H, m), 6.73 (1H, dd), 6.84 (1H, d), 7.19 (1H, d) |

(continued)

| Intermediate | Name (M+H) | $^1H$ NMR δ ($CDCl_3$) |
|---|---|---|
| 8 | 4-(3,4-Dichlorobenryl)-1-(piperidin-4-ylmethyl) piperidine (341/343) | 7.33 (1H, d), 7.23 (1H, s), 6.96 (1H, d), 3.06 (2H, d), 2.83 (2H, d), 2.58 (2H, t), 2.47 (2H, d), 2.11 (2H, d), 1.84 - 1.68 (4H, m), 1.64 - 1.52 (3H, m), 1.51 - 1.41 (1H, m), 1.31 - 1.21 (2H, m), 1.12 - 1.02 (2H, m) |
| 9 | 2-Chloro-3-methyl-4-{[1-(piperidin-4-ylmethyl) piperidin-4-yl]oxy}benzonitrile (347/349) | 1.07 - 1.23 (2H, m), 1.64 - 1.91 (5H, m), 2.01 - 2.11 (2H, m), 2.25 (2H, d), 2.34 (3H, s), 2.37 - 2.47 (2H, m), 2.57 - 2.75 (4H, m), 3.02 - 3.11 (2H, m), 4.58 - 4.66 (1H, m), 7.09 (1H, d), 7.62 (1H, d) |
| 10 | 4-(4-Chlorophenoxy)-1-(piperidin-4-ylmethyl) piperidine (309/311; retention time 1.66, standard) | |
| 11 | 3-Chloro-4-[[1-(piperidin-4-ylmethyl)-4-piperidinyl]oxy]-benzonitrile (333/335) | 1.21 - 1.32 (2H, m), 1.74 - 1.90 (5H, m), 1.99 - 2.10 (2H, m), 2.26 (2H, d), 2.31 - 2.40 (2H, m), 2.67 - 2.79 (4H, m), 3.11 - 3.21 (2H, m), 4.52 - 4.62 (1H, m), 7.05 (1H, dd), 7.21 (1H, d), 7.70 (1H, d) |

Intermediate 12

This illustrates the preparation of 2-chloro-4-(piperidin-4-yloxy)benzonitrile

4-Piperidinol (6.5 g) was added to a solution of potassium *tert*-butoxide (7.2 g) in N-methyl-2-pyrrolidinone (25 mL) and stirred at room temperature for 30 min. The reaction mixture was cooled to 0 °C and 2-chloro-4-fluoro-benzonitrile (10 g) was added portionwise. The reaction mixture was allowed to reach room temperature and was stirred overnight. LC/MS showed a major peak with the required product mass. The reaction was quenched by addition of ammonium chloride solution and the mixture was partitioned partitioned between ethyl acetate and water. The ethyl acetate was washed with water then brine, dried ($MgSO_4$), filtered and concentrated *in vacuo*. Crude material was purified by flash chromatography, eluting with 2 - 5% methanol, 0.1% triethylamine in dichloromethane to give the title compound (6.6 g).
MS (APCI+ve) 236/238
$^1H$ NMR δ($CD_3OD$) 2.00 - 2.29 (4H, m), 3.22 - 3.47 (4H, m), 4.85 - 4.92 (1H, m), 7.14 (1H, dd), 7.33 (1H, d), 7.76 (1H, d).

The following Intermediate was prepared analogously from 2-chloro-4-fluoro-3-methylbenzonitrile

| Intermediate | Name (M+H) | $^1H$ NMR δ ($CD_3OD$) |
|---|---|---|
| 13 | 2-chloro-3-methyl-4-(piperidin-4-yloxy) benzonitrile (Retention time 1.62, standard; 251/253) | 1.58 - 1.68 (2H, m), 1.89 - 1.98 (2H, m), 2.22 (3H, s), 2.63 - 2.72 (2H, m), 2.94 - 3.01 (2H, m), 4.53 - 4.62 (1H, m), 6.99 (1H, d), 7.49 (1H, d), |

Intermediate 14

This illustrates the preparation of 2-chloro-4-fluoro-3-methylbenzonitrile

2-Chloro-4-fluorobenzonitrile (1g) and TMEDA (1.13 mL) in THF (10 mL) were cooled to -78 °C, under nitrogen. *Sec*-butyllithium (1.3M in cyclohexane, 8.54 mL) was added over 20min, keeping the temperature below -70 °C. The mixture was then stirred at -78 °C for 2.5h. Methyl iodode (0.5mL) was added and the mixture allowed to warm to 15 °C, over 35min. The reaction was quenched with a saturated aqueous solution of ammonium chloride and the product was extracted with ethyl acetate. The ethyl acetate was washed with brine, dried (MgSO4), filtered and concentrated under reduced pressure to give crude product (1.28g).
Retention time 2.12 min (standard)

EXAMPLE 1

This Example illustrates the preparation of methyl 2-[4-[[4-(3,4-dichlorophenoxy)-1-piperidinyl]methyl]-1-piperidinyl]-benzoate

4-(3,4-Dichlorophenoxy)-1-(piperidin-4-ylmethyl)-piperidine (0.2 g) and methyl 2-fluorobenzoate (0.08 g) were dissolved in DMF (1 mL) and to the solution was added potassium carbonate (0.1 g). The reaction mixture was heated at 85°C overnight then poured into water and extracted with ethyl acetate. The ethyl acetate was washed with brine, dried ($MgSO_4$), filtered and concentrated *in vacuo* to give a crude yellow oil. This was purified by RPHPLC to give the title compound (0.15 g).

$^1H$ NMR δ($CD_3OD$) 1.36 - 1.51 (2H, m), 1.66 - 1.89 (5H, m), 1.99 - 2.09 (2H, m), 2.33 - 2.46 (4H, m), 2.70 - 2.84 (4H, m), 3.26 - 3.30 (2H, m), 3.83 (3H, s), 4.38 - 4.48 (1H, m), 6.92 (1H, dd), 7.00 (1H, t), 7.11 - 7.15 (2H, m), 7.38 - 7.47 (2H, m), 7.64 (1H, dd)

MS (APCI+ve) 476/478

Examples 2-21 in Table 1 were prepared analogously to Example 1 using the appropriate arylfluoride and amine substrates following the general method of Example 1.

Example 22

Methyl 2-(4-{[4-(3,4-dichlorophenoxy)piperidin-1-yl]methyl}piperidin-1-yl)nicotinate

Methyl 2-chloronicotinate (202 mg), 4-(3,4-Dichlorophenoxy)-1-(4-piperidinylmethyl)-piperidine (230 mg) and MP carbonate (Argonaut Technologies Inc) (412 mg) were combined in dimethylacetamide (4 ml) and heated to 120 °C for 5 minutes in a microwave oven (CEM discover). The mixture was filtered and added to water and ethyl acetate. The phases were separated, and the aqueous layer was extracted twice with ethyl acetate. The organic phases were washed with brine, water and brine again, they were then dried, filtered and evaporated. The residue was chromatographed eluting with dichloromethane : methanol (49:1) to give the title compound (125mg).

MS (ES+ve) 478 / 480 (M+H)+; retention time 2.79 (standard).

Example 23

Ethyl 3-(4-{[4-(3,4-dichlorophenoxy)piperidin-1-yl]methyl}piperidin-1-yl)benzoate

A mixture of 4-(3,4-dichlorophenoxy)-1-(4-piperidinylmethyl)-piperidine (350 mg), ethyl 3-iodobenzoate (161mg), tris-(dibenzylidineacetone)dipalladium (4.2 mg), caesium carbonate (378 mg), 2-dicyclohexylphosphino-2'-(N, N-dimethylamino)biphenyl (7 mg) in 2-methylpropan-2-ol (7 ml) and dioxan (7 ml) was heated to 95 °C for 1 h. Additional ethyl 3-iodobenzoate (138 mg) was added and heating was continued for 3 h. The mixture was allowed to cool and was then poured into water; the resultant mixture was extracted thrice with ethyl acetate, the organic extracts were combined, washed with brine, dried, filtered and evaporated. Chromatography of the residue (eluent 1:1 isohexane : ethyl acetate) gave the title compound (336 mg).

MS (ES+ve) 491/493 $(M+H)^+$

Examples 24-32 in Table 1 were prepared analogously to Example 23 using the appropriate aryl bromide or iodide and amine substrates.

EXAMPLE 33

This Example illustrates the preparation of 2-[4-[[4-(3,4-dichlorophenoxy)-1-piperidinyl]methyl]-1-piperidinyl]-benzoic acid

Methyl 2-[4-[[4-(3,4-dichlorophenoxy)-1-piperidinyl]methyl]-1-piperidinyl]-benzoate (0.11 g) was dissolved in THF (1 mL) and treated with potassium trimethylsilanolate (36 mg). The reaction mixture was stirred at room temperature overnight then diluted with methanol and acidified to pH 5 by addition of a few drops of acetic acid. The crude solution was purified by RPHPLC to give the title compound (0.080g).

$^1H$ NMR δ($CD_3OD$) 1.40 - 1.56 (2H, m), 1.64 - 1.76 (2H, m), 1.87 - 1.99 (3H, m), 2.06 (2H, d), 2.28 - 2.38 (4H, m), 2.67 - 2.76 (2H, m), 3.25 - 3.32 (4H, m), 4.28 - 4.38 (1H, m), 6.80 (1H, dd), 7.01 (1H, d), 7.29 (1H, d), 7.38 - 7.44 (1H, m), 7.54 - 7.64 (2H, m), 8.08 -8.10 (1H, m)

MS (APCI+ve) 460/462

The Examples in Table 2 were prepared analogously to Example 33 by hydrolysis of the corresponding ester using a standard method. In some cases the esters were not isolated and are not included in the table above; such esters were prepared by analogous methods to those in the table above. In the case of Examples 38 and 94 the nitrile in the starting ester also hydrolysed under the reaction conditions.

Table 1

| Example No. | Name | retention time | HPLC system ester | M+H | $^1$H NMR |
|---|---|---|---|---|---|
| 2 | Methyl 2-(4-{[4-(3-chloro-4-cyanophenoxy) piperidin-1-yl] methyl}piperidin-1-yl)benzoate | | | 466/468 | ($CD_3OD$) 1.35 - 1.50 (2H, m), 1.53 - 1.74 (2H, m), 1.76 - 1.90 (4H, m), 2.00 - 2.12 (1H, m), 2.26 - 2.43 (2H, m), 2.69 - 2.80 (4H, m), 3.25 - 3.30 (4H, m), 3.85 (3H, s), 4.53 - 4.62 (1H, m), 6.99 (1H, t), 7.06 (1H, d), 7.13 (1H, d), 7.21 (1H, d), 7.43 (1H, t), 7.64 (1H, d), 7.70 (1H, d) |
| 3 | Methyl 2-(4-{[4-(4-fluorobenzyl) piperidin-1-yl] methyl}piperidin-1-yl)benzoate | | | 425/427 | |
| 4 | Methyl 4-chloro-2-(4-{[4-(3-chloro-4-cyanophenoxy) piperidin-1-yl] methyl}piperidin-1-yl)benzoate | | | 502/504 +ve | $\delta_{(CD3OD)}$ 1.33 - 1.51 (3H, m), 1.52 - 1.89 (6H, m), 2.00 - 2.11 (2H, m), 2.29 - 2.43 (3H, m), 2.69 - 2.81 (4H, m), 3.48 (1H, d), 3.89 (3H, s), 4.51 - 4.63 (1H, m), 6.97 (1H, dd), 7.01 - 7.09 (2H, m), 7.19 - 7.22 (1H, m), 7.68 (2H, dd) |
| 5 | Methyl 2-[4-({4-[4-cyano-2-chlorophenoxy] piperidin-1-yl} methyl)piperidin-1-yl]benzoate | | | 468/470 +ve | $\delta_{(CD3OD)}$ 1.35 - 1.50 (2H, m), 1.63 - 1.79 (1H, m), 1.82 - 1.96 (5H, m), 2.01 - 2.13 (3H, m), 2.34 (2H, d), 2.41 - 2.51 (2H, m), 2.70 - 2.81 (4H, m), 3.86 (3H, s), 4.65 - 4.74 (1H, m), 6.96 - 7.04 (1H, m), 7.13 (1H, d), 7.28 (1H, dd), 7.39 - 7.48 (1H, m), 7.62 - 7.68 (2H, m), 7.81 (1H, t) |
| 6 | Ethyl 4-(4-{[4-(3,4-dichlorophenoxy) piperidin-1-yl] methyl}piperidin-1-yl)benzoate | | | 491/493 | |

(continued)

| Example No. | Name | retention time | HPLC system ester | M+H | $^1$H NMR |
|---|---|---|---|---|---|
| 7 | Methyl 4-chloro-2-(4-{[4-(2-chloro-4-cyanophenoxy) piperidin-1-yl] methyl}piperidin-1-yl)benzoate | | | 500/502 -ve | $\delta_{(CD3OD)}$ 1.34 - 1.45 (2H, m), 1.64 - 1.76 (1H, m), 1.81 - 1.91 (4H, m), 2.00 - 2.09 (2H, m), 2.30 (2H, d), 2.39 - 2.47 (2H, m), 2.68 - 2.78 (4H, m), 3.25 - 3.28 (2H, m), 3.87 (3H, s), 4.64 - 4.71 (1H, m), 6.94 (1H, dd), 7.05 (1H, d), 7.26 (1H, d), 7.61 - 7.66 (2H, m), 7.77 (1H, d) |
| 8 | Methyl 3-chloro-4-(4-{[4-(3-chloro-4-cyanophenoxy) piperidin-1-yl] methyl}piperidin-1-yl)benzoate | 3.02 | standard | 502/504 | |
| 9 | Methyl 2-chloro-4-(4-{[4-(3-chloro-4-cyanophenoxy) piperidin-1-yl] methyl}piperidin-1-yl)benzoate | 3.02 | standard | 502/504 | |
| 10 | Methyl 2-(4-{[4-(3,4-dichlorophenoxy) piperidin-1-yl] methyl}piperidin-1-yl)-4-methyl benzoate | 3.16 | standard | 491/493 | |
| 11 | 2-(4-{[4-(4-chlorophenoxy) piperidin-1-yl] methyl}piperidin-1-yl)benzoate | 2.64 | standard | | |
| 12 | Methyl 3-chloro-2-(4-{[4-(3-chloro-4-cyanophenoxy) piperidin-1-yl] methyl}piperidin-1-yl)benzoate | 2.9 | standard | 457/459 | |
| 13 | Methyl 2-(4-{[4-(3,4-dichlorophenoxy) piperidin-1-yl] methyl}-4-hydroxypiperidin-1-yl)benzoate | 3.18 | standard | 493/495 | |

(continued)

| Example No. | Name | retention time | HPLC system ester | M+H | $^1$H NMR |
|---|---|---|---|---|---|
| 14 | Methyl 2-(4-{[4-(4-fluorobenzyl) piperidin-1-yl] methyl}piperidin-1-yl)-5-methylbenzoate | 2.33 | standard | 439/441 | |
| 15 | Methyl 2-(4-{[3-(3,4-dichlorophenoxy) azetidin-1-yl] methyl}piperidin-1-yl)-5-fluorobenzoate | | | 437/439 -ve | $\delta_{(CD3OD)}$ 1.29 - 1.57 (3H, m), 1.79 (2H, d), 2.55 (2H, d), 2.70 (2H, t), 3.17 - 3.28 (4H, m), 3.83 - 3.91 (5H, m), 4.81 - 4.85 (1H, m), 6.81 (1H, dd), 7.02 (1H, d), 7.15 - 7.21 (2H, m), 7.35 - 7.40 (1H, m), 7.43 (1H, d) |
| 16 | Methyl 2-(4-{[3-(3,4-dichlorophenoxy) azetidin-1-yl] methyl}piperidin-1-yl)benzoate | | | 419/421 -ve | $\delta_{(CD3OD)}$ 1.35 - 1.57 (3H, m), 1.75 - 1.84 (2H, m), 2.55 (2H, d), 2.73 (2H, t), 3.21 - 3.28 (4H, m), 3.83 - 3.90 (5H, m), 4.80 - 4.86 (1H, m), 6.81 (1H, dd), 6.96 - 7.03 (2H, m), 7.12 (1H, dd), 7.40 - 7.47 (2H, m), 7.64 (1H, dd) |
| 17 | Methyl 2-(4-{[(3S)-3-(4-chlorophenoxy) pyrrolidin-1-yl] methyl}piperidin-1-yl)benzoate | | | 427/429 -ve | $\delta_{(CD3OD)}$ 1.35 - 1.51 (2H, m), 1.57 - 1.71 (1H, m), 1.82 - 2.00 (3H, m), 2.28 - 2.62 (4H, m), 2.69 - 2.97 (5H, m), 3.25 - 3.30 (2H, m), 3.89 (3H, s), 4.86 - 4.92 (1 H, m), 6.88 (2H, dd), 6.99 (1 H, t), 7.12 (1H, dd), 7.26 (2H, dd), 7.43 (1H, t), 7.64 (1H, d) |

(continued)

| Example No. | Name | retention time | HPLC system ester | M+H | $^1$H NMR |
|---|---|---|---|---|---|
| 18 | Methyl 2-(4-{[(3S)-3-(4-chlorophenoxy) pyrrolidin-1-yl] methyl}piperidin-1-yl)-5-fluorobenzoate | | | 445/447 -ve | $\delta_{(CD3OD)}$ 1.34 - 1.49 (2H, m), 1.56 - 1.70 (1H, m), 1.81 - 2.00 (3H, m), 2.28 - 2.62 (4H, m), 2.67 - 2.96 (5H, m), 3.18 - 3.27 (2H, m), 3.90 (3H, s), 4.86 - 4.92 (1H, m), 6.88 (2H, dd), 7.16 - 7.21 (2H, m), 7.26 (2H, dd), 7.35 - 7.39 (1H, m) |
| 19 | Methyl 2-(4-{[3-(3-chloro-4-cyanophenoxy) azetidin-1-yl] methyl}piperidin-1-yl)benzoate | | | 438/440 -ve | $\delta_{(CO3OD)}$ 1.30 - 1.59 (3H, m), 1.80 (2H, d), 2.54 (2H, d), 2.73 (2H, t), 3.24 - 3.32 (3H, m), 3.83 - 3.90 (5H, m), 4.92 - 5.01 (2H, m), 6.94 - 7.03 (2H, m), 7.10 - 7.14 (2H, m), 7.43 (1H, dd), 7.64 (1H, dd), 7.74 (1H, d) |
| 20 | Methyl 2-(4-{[4-(3-chloro-4-cyano-2-methylphenoxy) piperidin-1-yl] methyl}piperidin-1-yl)benzoate | 2.29min | standard gradient | 480/482 -ve | |
| 21 | Methyl 2-(4-{[3-(3-chloro-4-cyanophenoxy) azetidin-1-yl] methyl}piperidin-1-yl)-5-fluorobenzoate | 2.64min | standard gradient | 456/458 -ve | |
| 24 | Ethyl 3-(4-{[4-(3,4-dichloro-2-methylphenoxy) piperidin-1-yl] methyl}piperidin-1-yl)benzoate | | | 505/509 | |
| 25 | 1,1Dimethylethyl 3-(4-{[4-(3,4-dichlorophenoxy) piperidin-1- l] methyl}piperidin-1-yl)-4-methylbenzoate | 3.22 | fast | 533/535 | |

(continued)

| Example No. | Name | retention time | HPLC system ester | M+H | $^1$H NMR |
|---|---|---|---|---|---|
| 26 | 1,1Dimethylethyl 4-chloro-3-(4-{[4-(3,4-dichlorophenoxy) piperidin-1-yl] methyl}piperidin-1-yl)benzoate | 3.35 | fast | 555/557 | |
| 27 | 1,1Dimethylethyl 3-(4-{[4-(3,4-dichlorophenoxy) piperidin-1-yl] methyl}piperidin-1-yl)-4-methoxybenzoate | 2.63 | fast | 549/551 | |
| 28 | 1,1Dimethylethyl 3-(4-{[4-(2,4-dichloro-3-methylphenoxy) piperidin-1-yl] methyl}piperidin-1-yl)benzoate | | | 505/507 | |
| 29 | 1,1Dimethylethyl 2-chloro-5-(4-{[4-(3,4-dichlorophenoxy) piperidin-1-yl] methyl}piperidin-1-yl)benzoate | 2.91 | fast | 553/555/557 | |
| 30 | 1,1Dimethylethyl 6-(4-{[4-(3,4-dichlorophenoxy) piperidin-1-yl] methyl}piperidin-1-yl)pyridine-2-carboxylate | 2.43 | fast | 520/522 | |
| 31 | 1,1Dimethylethyl 5-(4-{[4-(3,4-dichlorophenoxy) piperidin-1-yl] methyl}piperidin-1-yl)-2-ethylnicotinate | 1.90 | fast | 534/536 | |
| 32 | Ethyl3-(4-{[4-(3,4-dichlorobenzyl) piperidin-1-yl] methyl}piperidin-1-yl)benzoate | 1.43 | fast | 489/491 | |

Table 2

| | | | | | | |
|---|---|---|---|---|---|---|
| Hydrolysis methods: | | | | | | |
| A, LiOH / aq THF or aq MeOH or aq THF - MeOH | | | | | | |
| B, KOTMS / THF / RT 3 days | | | | | | |
| C, TFA/ dichloromethane | | | | | | |
| D, 6M HCl aq reflux | | | | | | |
| E, 4M HCl / dioxan | | | | | | |
| Example | Name | Hydrolysis Method | MS $(M+H)^+$ ES +ve | retention time | HPLC system | $^1H$ NMR $\delta_{(CD3OD+1drop\ N\alpha O\Delta)}$ |
| 34 | 2-(4-{[4-(3-chloro-4-cyanophenoxy) piperidin-1-yl]methyl} piperidin-1-yl)benzoic acid | A | 452/454 -ve | | | 1.52 - 1.68 (2H, m), 1.78 - 1.91 (2H, m), 1.98 - 2.22 (5H, m), 2.43 (2H, d), 2.45 - 2.52 (2H, m), 2.78 - 2.88 (2H, m), 3.36 - 3.43 (4H, m), 4.55 - 4.65 (1H, m), 7.06 (1H, dd), 7.22 (1H, d), 7.54 (1H, dd), 7.67 - 7.76 (3H, m), 8.19 - 8.22 (1H, m) |
| 35 | 2-(4-{[4-(4-fluorobenzyl)piperidin-1-yl]methyl}piperidin-1-yl)benzoic acid | A | 411/413 | | | 1.27 - 1.45 (3H, m), 1.50 - 1.75 (5H, m), 1.97 - 2.13 (3H, m), 2.26 (2H, s), 2.51 - 2.59 (4H, m), 3.10 - 3.17 (2H, m), obscured: 3.32 - 3.39 (3H, m), 6.96 - 7.03 (2H, m), 7.14 - 7.20 (2H, m), 7.43 - 7.49 (1H, m), 7.64 (2H, d), 8.12(1H, d) |
| 36 | 4-chloro-2-(4-{[4-(3-chloro-4-cyanophenoxy) piperidin-1-yl]methyl} piperidin-1-yl)benzoic acid | A | 488 / 490 | | | 1.49 - 1.69 (2H, m), 1.86 - 2.24 (9H, m), 2.57 - 2.78 (4H, m), 2.92 - 3.07 (2H, m), 3.15 - 3.30 (2H, m), 4.64 - 4.76 (1H, m), 7.05 - 7.15 (1H, m), 7.23 - 7.31 (1H, m), 7.40 - 7.49 (1H, m), 7.67 - 7.79 (2H, m), 8.01 - 8.10 (1H, m) |

(continued)

| Hydrolysis methods: | | | | | | |
|---|---|---|---|---|---|---|
| A, LiOH / aq THF or aq MeOH or aq THF - MeOH | | | | | | |
| B, KOTMS / THF / RT 3 days | | | | | | |
| C, TFA/ dichloromethane | | | | | | |
| D, 6M HCl aq reflux | | | | | | |
| E, 4M HCl / dioxan | | | | | | |
| Example | Name | Hydrolysis Method | MS $(M+H)^+$ ES +ve | retention time | HPLC system | $^1H$ NMR $\delta_{(CD3OD+1drop\ N\alpha O\Delta)}$ |
| 37 | 2-[4-({4-[4-(aminocarbonyl)-2-chlorophenoxy]piperidin-1-yl}methyl)piperidin-1-yl]benzoic acid | A | 472 / 474 | | | 1.39 - 1.51 (2H, m), 1.58 - 1.70 (1H, m), 1.76 - 1.91 (4H, m), 1.99 - 2.08 (2H, m), 2.29 (2H, d), 2.37 - 2.45 (2H, m), 2.64 - 2.77 (4H, m), 3.46 - 3.51 (2H, m), 4.58 - 4.66 (1H, m), 6.85 (1H, td), 6.95 (1H, d), 7.13 -7.19 (2H, m), 7.32 (1H, dd), 7.80 (1H, dd), 7.94 (1H, d) |
| 38 | 4-(4-{[4-(3,4-dichlorophenoxy)piperidin-1-yl]methyl}piperidin-1-yl)benzoic acid | A | 463 / 465 (M+H) | | | 1.28 - 1.42 (2H, m), 1.73 - 1.86 (3H, m), 1.91 (2H, d), 2.00 - 2.10 (2H, m), 2.30 (2H, d), 2.38 (2H, t), 2.72 - 2.84 (4H, m), 3.83 (2H, d), 4.44 (1H, dd), 6.94 (3H, dd), 7.11 (1H, d), 7.42 (1H, d), 7.88 (2H, d) |
| 39 | 4-chloro-2-(4-{[4-(2-chloro-4-cyanophenoxy)piperidin-1-yl]methyl}piperidin-1-yl)benzoic acid | A | 486 / 488 -ve | | | 1.48 - 1.64 (2H, m), 1.91 - 2.20 (7H, m), 2.55 (2H, d), 2.64 - 2.75 (2H, m), 2.87 - 2.98 (2H, m), 3.15 - 3.27 (2H, m), 3.35 - 3.41 (2H, m), 4.72 - 4.82 (1H, m), 7.30 (1H, d), 7.42 (1H, d), 7.65 - 7.70 (2H, m), 7.82 (1H, dd), 8.02 (1H, d) |

(continued)

| Hydrolysis methods: | | | | | | |
|---|---|---|---|---|---|---|
| A, LiOH / aq THF or aq MeOH or aq THF - MeOH | | | | | | |
| B, KOTMS / THF / RT 3 days | | | | | | |
| C, TFA/ dichloromethane | | | | | | |
| D, 6M HCl aq reflux | | | | | | |
| E, 4M HCl / dioxan | | | | | | |
| Example | Name | Hydrolysis Method | MS $(M+H)^+$ ES +ve | retention time | HPLC system | $^1H$ NMR $\delta_{(CD3OD+1drop\ N\alpha O\Delta)}$ |
| 40 | 3-chloro-4-(4-{[4-(3-chloro-4-cyanophenoxy) piperidin-1-yl]methyl} piperidin-1- yl)benzoic acid | B | 488 / 490 | 1.28 | Standard | 1.36 - 1.50 (2H, m), 1.63 - 1.91 (5H, m), 1.98 - 2.10 (2H, m), 2.27 - 2.42 (4H, m), 2.62 - 2.81 (4H, m), 3.36 - 3.46 (2H, m), 4.51 - 4.60 (1H, m), 7.04 (1H, dd), 7.07 (1H, d), 7.19 (1H, d), 7.69 (1H, d), 7.80 (1H, dd), 7.92 (1H, d) |
| 41 | 2-chloro-4-(4-{[4-(3-chloro- 4-cyanophenoxy) piperidin-1-yl]methyl} piperidin-1-yl)benzoic acid | B | | 1.17 | Standard | 1.15 - 1.27 (2H, m), 1.58 - 1.82 (5H, m), 1.90 - 1.99 (2H, m), 2.17 (2H, d), 2.22 - 2.31 (2H, m), 2.58 - 2.69 (4H, m), 3.59 - 3.67 (2H, m), 4.42 - 4.50 (1H, m), 6.74 (1H, dd), 6.78 (1H, d), 6.94 (1H, dd), 7.09 (1H, d), 7.34 (1H, d), 7.59 (1H, d) |
| 42 | 2-(4-{[4-(3,4-dichlorophenoxy) piperidin-1-yl]methyl} piperidin-1-yl)-4-methylbenzoic acid | B | 477 / 479 | 2.07 | Standard | 1.28 - 1.41 (2H, m), 1.46 - 1.5 (1H, m), 1.62 - 1.73 (4H, m), 1.86 - 1.96 (2H, m), 2.12 - 2.28 (7H, m), 2.52 - 2.60 (2H, m), 2.60 - 2.69 (2H, m), 3.33 - 3.40 (2H, m), 4.25 - 4.33 (1H, m), 6.57 (1 H, d), 6.65 (1H, s), 6.79 (1 H, dd), 7.00 (1 H, d), 7.14 (1H, d), 7.28 (1H, d) |

(continued)

| Hydrolysis methods: | | | | | | |
|---|---|---|---|---|---|---|
| A, LiOH / aq THF or aq MeOH or aq THF - MeOH | | | | | | |
| B, KOTMS / THF / RT 3 days | | | | | | |
| C, TFA/ dichloromethane | | | | | | |
| D, 6M HCl aq reflux | | | | | | |
| E, 4M HCl / dioxan | | | | | | |
| Example | Name | Hydrolysis Method | MS $(M+H)^+$ ES +ve | retention time | HPLC system | $^1H$ NMR $\delta$(CD3OD+1drop NαOΔ) |
| 43 | 2-(4-{[4-(4-chlorophenoxy) piperidin-1-yl]methyl} piperidin-1-yl)-4-methylbenzoic acid | B | 443 / 445 | 1.84 | Standard | 1.37 - 1.55 (2H, m), 1.58 - 1.70 (1H, m), 1.74 - 1.86 (4H, m), 1.97 - 2.09 (2H, m), 2.25 - 2.39 (7H, m), 2.62 - 2.84 (4H, m), 3.43 - 3.54 (2H, m), 4.32 - 4.43 (1H, m), 6.66 - 6.80 (2H, m), 6.89 - 6.98 (2H, m), 7.21 - 7.31 (3H, m) |
| 44 | 2-(4-{[4-(4-chlorophenoxy) piperidin-1-yl]methyl} piperidin-1-yl)benzoic acid | B | | 2.64 | Standard | 1.39 - 1.56 (2H, m), 1.58 - 1.72 (1H, m), 1.75 - 1.87 (4H, m), 1.96 - 2.09 (2H, m), 2.26 - 2.41 (4H, m), 2.64 - 2.83 (4H, m), 3.45 - 3.57 (2H, m), 4.33 - 4.43 (1H, m), 6.82 - 6.99 (4H, m), 7.13 - 7.37 (4H, m) |
| 45 | 2-(4-{[4-(3-chloro-4-cyanophenoxy) piperidin-1-yl]methyl} piperidin-1-yl)-6-(trifluoromethyl) nicotinic acid | A | 523 / 525 | 1.52 | Standard | 1.23 - 1.37 (2H, m), 1.71 - 1.86 (4H, m), 1.98 - 2.09 (2H, m), 2.24 (2H, d), 2.30 - 2.40 (2H, m), 2.70 (2H, s), 2.92 (2H, t), 3.30 (1H, s), 4.14 (2H, d), 4.51 - 4.58 (1H, m), 6.96 (1H, d), 7.04 (1H, dd), 7.14 (1H, d), 7.67 - 7.75 (2H, m) |

(continued)

| Hydrolysis methods: | | | | | | |
|---|---|---|---|---|---|---|
| A, LiOH / aq THF or aq MeOH or aq THF - MeOH | | | | | | |
| B, KOTMS / THF / RT 3 days | | | | | | |
| C, TFA/ dichloromethane | | | | | | |
| D, 6M HCl aq reflux | | | | | | |
| E, 4M HCl / dioxan | | | | | | |
| Example | Name | Hydrolysis Method | MS $(M+H)^+$ ES +ve | retention time | HPLC system | $^1$H NMR $\delta$(CD3OD+1drop NαOΔ) |
| 46 | 2-(4-{[4-(3-chloro-4-cyanophenoxy) piperidin-1-yl]methyl} piperidin-1-yl)-5-nitrobenzoic acid | A | 499 / 501 | | | 1.33 - 1.52 (2H, m), 1.76 - 1.89 (5H, m), 2.00 - 2.11 (2H, m), 2.28 - 2.44 (4H, m), 2.71 - 2.81 (2H, m), 2.93 - 3.05 (2H, m), 3.75 - 3.85 (2H, m), 4.52 - 4.64 (1H, m), 6.98 (1 H, d), 7.06 (1H, dd), 7.21 (1 H, d), 7.71 (1H, d), 8.05 (1H, dd), 8.24 (1H, d) |
| 47 | 2-(4-{[4-(3,4-dichlorophenoxy) piperidin- 1-yl]methyl} piperidin-1-yl)- -methylnicotinic acid | A | 478 / 476 | 1.75 | standard | 1.35 (2H, q), 1.69 - 1.82 (5H, m), 1.96 - 2.04 (2H, m), 2.25 (2H, d), 2.29 - 2.37 (2H, m), 2.34 (3H, s), 2.68 - 2.77 (2H, m), 2.87 (2H, t), 3.98 (2H, d), 4.33 - 4.43 (1H, m), 6.54 (1H, d), 6.86 - 6.91 (1H, m), 7.07 (1H, d), 7.37 (1H, d), 7.56 (1H, d) |
| 48 | 3-chloro-2-(4-{[4-(3-chloro- 4-cyanophenoxy) piperidin-1-yl]methyl} piperidin-1-yl)benzoic acid | A | 488 / 490 | | | 1.21 - 1.36 (2H, m), 1.55 - 1.77 (5H, m), 1.89 - 2.00 (2H, m), 2.16 - 2.31 (4H, m), 2.59 - 2.69 (2H, m), 3.06 - 3.13 (4H, m), 4.41 - 4.50 (1H, m), 6.83 (1H, t), 6.94 (1H, dd), 7.07 - 7.10 (2H, m), 7.15 (1H, dd), 7.59 (1H, d) |

(continued)

| Hydrolysis methods: | | | | | | |
|---|---|---|---|---|---|---|
| A, LiOH / aq THF or aq MeOH or aq THF - MeOH | | | | | | |
| B, KOTMS / THF / RT 3 days | | | | | | |
| C, TFA/ dichloromethane | | | | | | |
| D, 6M HCl aq reflux | | | | | | |
| E, 4M HCl / dioxan | | | | | | |
| Example | Name | Hydrolysis Method | MS $(M+H)^+$ ES +ve | retention time | HPLC system | $^1H$ NMR $\delta_{(CD3OD+1drop\ N\alpha O\Delta)}$ |
| 49 | 2-(4-{[4-(4-chloro-2-methylphenoxy) piperidin-1-yl]methyl} piperidin-1-yl)benzoic acid | B | 442 / 444 | 1.99 | Standard | 1.34 - 1.51 (2H, m), 1.57 - 1.69 (1H, m), 1.74 - 1.86 (4H, m), 1.94 - 2.06 (2H, m), 2.18 (3H, s), 2.25 - 2.41 (4H, m), 2.62 - 2.77 (4H, m), 3.44 - 3.52 (2H, m), 4.34 - 4.42 (1H, m), 6.83 - 6.89 (2H, m), 6.93 - 6.96 (1H, m), 7.06 - 7.11 (2H, m), 7.13 - 7.18 (1H, m), 7.32 (1H, dd) |
| 50 | 2-(4-{[4-(3-chloro-4-cyanophenoxy) piperidin-1-yl]methyl} piperidin-1-yl)-4-(trifluoromethyl) benzoic acid | A | 522 / 524 | | | 1.39 - 1.54 (2H, m), 1.63 - 1.75 (1H, m), 1.79 - 1.89 (4H, m), 2.00 - 2.12 (2H, m), 2.31 (2H, d), 2.34 - 2.43 (2H, m), 2.71 - 2.81 (4H, m), 3.52 - 3.60 (2H, m), 4.53 - 4.62 (1H, m), 7.06 (1H, dd), 7.14 (2H, d), 7.21 (1H, d), 7.46 (1H, d), 7.71 (1H, d) |
| 51 | 5-chloro-2-(4-{[4-(3-chloro-4-cyanophenoxy) piperidin-1-yl]methyl} piperidin-1-yl)benzoic acid | A | 488/490 | | | 1.37 - 1.52 (2H, m), 1.57 - 1.72 (1H, m), 1.75 - 1.88 (4H, m), 2.00 - 2.10 (2H, m), 2.30 (2H, d), 2.37 (2H, t), 2.63 - 2.80 (4H, m), 3.43 - 3.51 (2H, m), 4.53 - 4.62 (1H, m), 6.93 (1H, d), 7.06 (1H, dd), 7.16 (1H, dd), 7.21 (1H, d), 7.30 (1H, d), 7.71 (1H, d) |

(continued)

| Hydrolysis methods: | | | | | | |
|---|---|---|---|---|---|---|
| A, LiOH / aq THF or aq MeOH or aq THF - MeOH | | | | | | |
| B, KOTMS / THF / RT 3 days | | | | | | |
| C, TFA/ dichloromethane | | | | | | |
| D, 6M HCl aq reflux | | | | | | |
| E, 4M HCl / dioxan | | | | | | |
| Example | Name | Hydrolysis Method | MS $(M+H)^+$ ES +ve | retention time | HPLC system | $^1$H NMR $\delta_{(CD3OD+1drop\ N\alpha O\Delta)}$ |
| 52 | 2-(4-{[4-(3-chloro-4-cyanophenoxy) piperidin-1-yl]methyl} piperidin-1-yl)-5-(trifluoromethyl) benzoic acid | A | 522 / 524 | | | 1.38 - 1.53 (2H, m), 1.64 - 1.89 (5H, m), 2.01 - 2.12 (2H, m), 2.31 (2H, d), 2.38 (2H, t), 2.71 - 2.86 (4H, m), 3.59 - 3.67 (2H, m), 4.54 - 4.63 (1H, m), 7.01 - 7.08 (2H, m), 7.21 (1H, d), 7.44 (1H, dd), 7.62 (1H, d), 7.71 (1H, d) |
| 53 | 2-(4-{[4-(3-chloro-4-cyanophenoxy) piperidin-1-yl]methyl} piperidin-1-yl)-3-fluorobenzoic acid | A | 472 / 474 | | | 1.29 - 1.47 (2H, m), 1.59 - 1.88 (5H, m), 2.00 - 2.11 (2H, m), 2.29 (2H, d), 2.37 (2H, t), 2.71 - 2.80 (2H, m), 3.14 (2H, t), 3.23 - 3.32 (2H, m), 4.52 - 4.62 (1H, m), 6.87 - 7.09 (4H, m), 7.21 (1H, d), 7.71 (1H, d) |
| 54 | 2-(4-{[4-(3-chloro-4-cyanophenoxy) piperidin-1-yl]methyl} piperidin-1-yl)-5-fluorobenzoic acid | A | 472 / 474 | | | 1.37 - 1.52 (2H, m), 1.57 - 1.72 (1H, m), 1.76 - 1.88 (4H, m), 2.00 - 2.11 (2H, m), 2.30 (2H, d), 2.32 - 2.43 (2H, m), 2.61 - 2.81 (4H, m), 3.37 - 3.45 (2H, m), 4.52 - 4.62 (1H, m), 6.88 - 7.09 (4H, m), 7.21 (1H, d), 7.71 (1H, d) |

(continued)

| Hydrolysis methods: | | | | | | |
|---|---|---|---|---|---|---|
| A, LiOH / aq THF or aq MeOH or aq THF - MeOH | | | | | | |
| B, KOTMS / THF / RT 3 days | | | | | | |
| C, TFA/ dichloromethane | | | | | | |
| D, 6M HCl aq reflux | | | | | | |
| E, 4M HCl / dioxan | | | | | | |
| Example | Name | Hydrolysis Method | MS $(M+H)^+$ ES +ve | retention time | HPLC system | $^1$H NMR $\delta_{(CD3OD+1drop\ N\alpha O\Delta)}$ |
| 55 | 2-(4-{[4-(3-chloro-4-cyanophenoxy) piperidin-1-yl]methyl} piperidin-1-yl)-3-methoxybenzoic acid | A | 484 / 486 | | | 1.31 - 1.42 (2H, m), 1.56 - 1.72 (3H, m), 1.75 - 1.85 (2H, m), 1.99 - 2.07 (2H, m), 2.26 (2H, d), 2.31 - 2.39 (2H, m), 2.68 - 2.77 (2H, m), 3.11 - 3.21 (4H, m), 3.80 (3H, s), 4.52 - 4.59 (1H, m), 6.81 (2H, d), 6.97 (1H, t), 7.04 (1H, dd), 7.18 (1H, d), 7.69 (1H, d) |
| 56 | 2-(4-{[4-(3-chloro-4-cyanophenoxy) piperidin-1-yl]methyl} piperidin-1-yl)-5-methylbenzoic acid | A | 468 / 470 | | | 1.37 - 1.48 (2H, m), 1.56 - 1.65 (1H, m), 1.73 - 1.84 (4H, m), 1.99 - 2.08 (2H, m), 2.23 (3H, s), 2.27 (2H, d), 2.31 - 2.39 (2H, m), 2.63 (2H, dd), 2.69 - 2.77 (2H, m), 3.39 - 3.45 (2H, m), 4.52 - 4.59 (1H, m), 6.85 (1H, d), 6.98 (1H, dd), 7.01 - 7.05 (1H, m), 7.14 (1H, d), 7.18 (1H, d), 7.69 (1H, d) |
| 57 | 2-(4-{[4-(4-chloro-2-methylphenoxy) piperidin-1-yl]methyl} piperidin-1-yl)-3-fluorobenzoic acid | A | 461 / 463 | | | 1.32 - 1.48 (2H, m), 1.61 - 1.89 (5H, m), 1.97 - 2.08 (2H, m), 2.21 (3H, s), 2.29 (2H, d), 2.33 - 2.43 (2H, m), 2.67 - 2.78 (2H, m), 3.14 (2H, t), 3.23 - 3.32 (2H, m), 4.35 - 4.46 (1H, m), 6.87-7.14 (6H, m) |

(continued)

| | | | | | | |
|---|---|---|---|---|---|---|
| Hydrolysis methods: | | | | | | |
| A, LiOH / aq THF or aq MeOH or aq THF - MeOH | | | | | | |
| B, KOTMS / THF / RT 3 days | | | | | | |
| C, TFA/ dichloromethane | | | | | | |
| D, 6M HCl aq reflux | | | | | | |
| E, 4M HCl / dioxan | | | | | | |
| Example | Name | Hydrolysis Method | MS $(M+H)^+$ ES +ve | retention time | HPLC system | $^1$H NMR $\delta_{(CD3OD+1drop\ N\alpha O\Delta)}$ |
| 58 | 2-(4-{[4-(4-chloro-2-methylphenoxy) piperidin-1-yl]methyl} piperidin-1-yl)-5-(trifluoromethyl) benzoic acid | A | 511 / 513 | | | 1.38 - 1.53 (2H, m), 1.65 - 1.88 (5H, m), 1.97 - 2.08 (2H, m), 2.21 (3H, s), 2.30 (2H, d), 2.34 - 2.44 (2H, m), 2.68 - 2.87 (4H, m), 3.63 (2H, d), 4.36 - 4.45 (1H, m), 6.90 (1 H, d), 7.01 - 7.14 (3H, m), 7.44 (1H, dd), 7.62 (1H, d) |
| 59 | 2-(4-{[4-(4-chloro-2-methylphenoxy) piperidin-1-yl]methyl} piperidin-1-yl)-5-nitrobenzoic acid | A | 488 / 490 | | | 1.36 - 1.51 (2H, m), 1.77 - 1.89 (5H, m), 1.97 - 2.08 (2H, m), 2.20 (3H, s), 2.30 (2H, d), 2.33 - 2.45 (2H, m), 2.68 - 2.79 (2H, m), 2.94 - 3.05 (2H, m), 3.80 (2H, d), 4.37 - 4.45 (1H, m), 6.90 (1H, d), 6.99 (1H, d), 7.07 - 7.14 (2H, m), 8.06 (1H, dd), 8.24 (1H, d) |
| 60 | 2-(4-{[4-(4-chloro-2-methylphenoxy) piperidin-1-yl]methyl} piperidin-1-yl)-3-methoxybenzoic acid | A | 473 / 475 | | | 1.29 - 1.47 (2H, m), 1.57 - 1.76 (3H, m), 1.77 - 1.89 (2H, m), 1.97 - 2.08 (2H, m), 2.20 (3H, s), 2.28 (2H, d), 2.33 - 2.43 (2H, m), 2.67 - 2.78 (2H, m), 3.13 - 3.23 (4H, m), 3.82 (3H, s), 4.36 - 4.45 (1H, m), 6.84 (2H, d), 6.90 (1H, d), 6.99 (1 H, dd), 7.08 - 7.13 (2H, m) |

(continued)

| Hydrolysis methods: | | | | | | |
|---|---|---|---|---|---|---|
| A, LiOH / aq THF or aq MeOH or aq THF - MeOH | | | | | | |
| B, KOTMS / THF / RT 3 days | | | | | | |
| C, TFA/ dichloromethane | | | | | | |
| D, 6M HCl aq reflux | | | | | | |
| E, 4M HCl / dioxan | | | | | | |
| Example | Name | Hydrolysis Method | MS $(M+H)^+$ ES +ve | retention time | HPLC system | $^1$H NMR $\delta_{(CD3OD+1drop\ N\alpha O\Delta)}$ |
| 61 | 2-(4-{[4-(4-chloro-2-methylphenoxy) piperidin-1-yl]methyl} piperidin-1-yl)-4-(trifluoromethyl) benzoic acid | A | 511 / 513 | | | 1.40 - 1.56 (2H, m), 1.64 - 1.75 (1H, m), 1.79 - 1.89 (4H, m), 1.97 - 2.08 (2H, m), 2.21 (3H, s), 2.31 (2H, d), 2.35 - 2.44 (2H, m), 2.69 - 2.81 (4H, m), 3.56 (2H, d), 4.37 - 4.45 (1H, m), 6.90 (1H, d), 7.08 - 7.16 (4H, m), 7.46 (1H, d) |
| 62 | 4-chloro-2-(4-{[4-(4-chloro-2-methylphenoxy) piperidin-1-yl]methyl} piperidin-1-yl)benzoic acid | A | 477 / 479 | | | 1.38 - 1.49 (2H, m), 1.59 - 1.71 (1H, m), 1.75 - 1.85 (4H, m), 1.96 - 2.04 (2H, m), 2.18 (3H, s), 2.27 (2H, d), 2.32 - 2.39 (2H, m), 2.64 - 2.74 (4H, m), 3.45 - 3.52 (2H, m), 4.34 - 4.43 (1H, m), 6.81 (1H, dd), 6.86 - 6.89 (2H, m), 7.06 (1H, d), 7.08 - 7.10 (1H, m), 7.30 (1H, d) |
| 63 | 2-(4-{[4-(3,4-dichlorophenoxy) piperidin-1-yl]methyl}-4-hydroxypiperidin-1-yl)benzoic acid | B | 479 / 481 | | | 1.30 - 1.40 (1H, m), 1.69 (2H, d), 1.76 - 1.90 (4H, m), 1.96 - 2.06 (2H, m), 2.43 (2H, s), 2.52 - 2.60 (2H, m), 2.88 - 2.95 (2H, m), 3.05 - 3.13 (2H, m), 3.21 - 3.28 (2H, m), 4.35 - 4.43 (1H, m), 6.86 - 6.94 (2H, m), 7.01 (1H, d), 7.11 (1H, d), 7.18 - 7.24 (1H, m), 7.35 - 7.43 (2H, m) |

(continued)

| Hydrolysis methods: | | | | | | |
|---|---|---|---|---|---|---|
| A, LiOH / aq THF or aq MeOH or aq THF - MeOH | | | | | | |
| B, KOTMS / THF / RT 3 days | | | | | | |
| C, TFA/ dichloromethane | | | | | | |
| D, 6M HCl aq reflux | | | | | | |
| E, 4M HCl / dioxan | | | | | | |
| Example | Name | Hydrolysis Method | MS $(M+H)^+$ ES +ve | retention time | HPLC system | $^1H$ NMR $\delta_{(CD3OD+1drop\ N\alpha O\Delta)}$ |
| 64 | 2-(4-{[4-(4-chloro-2-methylphenoxy) piperidin-1-yl]methyl} piperidin-1-yl)-5-fluorobenzoic acid | A | 461 / 463 | | | 1.37 - 1.53 (2H, m), 1.57 - 1.71 (1H, m), 1.75 - 1.87 (4H, m), 1.96 - 2.08 (2H, m), 2.20 (3H, s), 2.29 (2H, d), 2.33 - 2.43 (2H, m), 2.61 - 2.77 (4H, m), 3.41 (2H, d), 4.36 - 4.45 (1H, m), 6.88 - 7.13 (6H, m) |
| 65 | 3-chloro-2-(4-{[4-(4-chloro-2-methylphenoxy) piperidin-1-yl]methyl} piperidin-1-yl)benzoic acid | A | 477 / 479 | | | 1.34 - 1.48 (2H, m), 1.64 - 1.88 (5H, m), 1.97 - 2.08 (2H, m), 2.20 (3H, s), 2.30 (2H, d), 2.33 - 2.44 (2H, m), 2.67 - 2.77 (2H, m), 3.19 - 3.26 (4H, m), 4.36 - 4.45 (1H, m), 6.87 - 6.98 (2H, m), 7.07 - 7.13 (2H, m), 7.18 - 7.30 (2H, m) |
| 66 | 5-chloro-2-(4-{[4-(4-chloro-2-methylphenoxy) piperidin-1-yl]methyl} piperidin-1- yl)benzoic acid | A | 477 / 479 | | | 1.37 - 1.53 (2H, m), 1.59 - 1.73 (1H, m), 1.77 - 1.88 (4H, m), 1.97 - 2.07 (2H, m), 2.20 (3H, s), 2.29 (2H, d), 2.33 - 2.43 (2H, m), 2.64 - 2.77 (4H, m), 3.44 - 3.51 (2H, m), 4.36 - 4.45 (1H, m), 6.92 (2H, dd), 7.07 - 7.19 (3H, m), 7.30 (1H, d) |

(continued)

| Hydrolysis methods: | | | | | | |
|---|---|---|---|---|---|---|
| A, LiOH / aq THF or aq MeOH or aq THF - MeOH | | | | | | |
| B, KOTMS / THF / RT 3 days | | | | | | |
| C, TFA/ dichloromethane | | | | | | |
| D, 6M HCl aq reflux | | | | | | |
| E, 4M HCl / dioxan | | | | | | |
| Example | Name | Hydrolysis Method | MS $(M+H)^+$ ES +ve | retention time | HPLC system | $^1H$ NMR $\delta_{(CD3OD+1drop\ N\alpha O\Delta)}$ |
| 67 | 2-(4-{[4-(4-chloro-3-methylphenoxy) piperidin-1- yl]methyl} piperidin-1-yl)-4-(trifluoromethyl) benzoic acid | A | 509/511 -ve | | | 1.40 - 1.55 (2H, m), 1.63 - 1.88 (5H, m), 1.96 - 2.07 (2H, m), 2.27 - 2.38 (7H, m), 2.70 - 2.81 (4H, m), 3.56 (2H, d), 4.32 - 4.41 (1H, m), 6.76 (1H, dd), 6.87 (1H, d), 7.11 - 7.17 (2H, m), 7.22 (1H, d), 7.46 (1 H, d) |
| 68 | 2-(4-{[4-(4-chloro-3-methylphenoxy) piperidin-1- yl]methyl} piperidin-1-yl)-3-luorobenzoic acid | A | 459/461 -ve | | | 1.32 - 1.47 (2H, m), 1.60 - 1.85 (5H, m), 1.96 - 2.07 (2H, m), 2.26 - 2.37 (7H, m), 2.71 - 2.81 (2H, m), 3.08 - 3.20 (2H, m), 3.23 - 3.31 (2H, m), 4.32 - 4.41 (1H, m), 6.73 - 6.79 (1H, m), 6.86 - 7.07 (4H, m), 7.20 - 7.25 (1H, m) |
| 69 | 2-(4-{[4-(4-chloro-3-ethylphenoxy) piperidin-1- yl]methyl} piperidin-1-yl)-5-fluorobenzoic acid | A | 459/461 -ve | | | 1.38 - 1.53 (2H, m), 1.57 - 1.70 (1H, m), 1.74 - 1.85 (4H, m), 1.96 - 2.07 (2H, m), 2.27 - 2.39 (7H, m), 2.61 - 2.81 (4H, m), 3.37 - 3.44 (2H, m), 4.32 - 4.42 (1H, m), 6.76 (1H, dd), 6.86 - 7.06 (4H, m), 7.22 (1H, d) |

(continued)

| Hydrolysis methods: | | | | | | |
|---|---|---|---|---|---|---|
| A, LiOH / aq THF or aq MeOH or aq THF - MeOH | | | | | | |
| B, KOTMS / THF / RT 3 days | | | | | | |
| C, TFA/ dichloromethane | | | | | | |
| D, 6M HCl aq reflux | | | | | | |
| E, 4M HCl / dioxan | | | | | | |
| Example | Name | Hydrolysis Method | MS $(M+H)^+$ ES +ve | retention time | HPLC system | $^1H$ NMR $\delta$(CD3OD+1drop NαOΔ) |
| 70 | 2-(4-{[4-(4-chloro-3-ethylphenoxy) piperidin-1- yl]methyl} piperidin-1-yl)-5-(trifluoromethyl) benzoic acid | A | 509 / 511 -ve | | | 1.39 - 1.55 (2H, m), 1.65 - 1.87 (5H, m), 1.96 - 2.07 (2H, m), 2.28 - 2.39 (7H, m), 2.71 - 2.87 (4H, m), 3.59 - 3.68 (2H, m), 4.31 - 4.42 (1H, m), 6.74 - 6.80 (1H, m), 6.86 - 6.89 (1H, m), 7.01 - 7.06 (1H, m), 7.20 - 7.25 (1H, m), 7.41 - 7.47 (1H, m), 7.60 - 7.63 (1H, m) |
| 71 | 2-(4-{[4-(4-chloro-2-methylphenoxy) piperidin-1-yl]methyl} piperidin-1-yl)-5-methylbenzoic acid | A | 455 / 457 -ve | | | 1.35 - 1.52 (2H, m), 1.55 - 1.69 (1H, m), 1.74 - 1.88 (4H, m), 1.95 - 2.07 (2H, m), 2.17 - 2.43 (10H, m), 2.57 - 2.74 (4H, m), 3.40 - 3.49 (2H, m), 4.34 - 4.44 (1H, m), 6.84 - 6.91 (2H, m), 6.98 - 7.04 (1 H, m), 7.05 - 7.11 (2H, m), 7.18 - 7.22 (1H, m) |
| 72 | 5-chloro-2-(4-{[4-(4-chloro-3-methylphenoxy) piperidin-1-yl]methyl} piperidin-1-yl)benzoic acid | A | 475 / 477 -ve | | | 1.38 - 1.53 (2H, m), 1.58 - 1.70 (1H, m), 1.74 - 1.85 (4H, m), 1.95 - 2.07 (2H, m), 2.27 - 2.39 (7H, m), 2.64 - 2.81 (4H, m), 3.43 - 3.52 (2H, m), 4.32 - 4.41 (1 H, m), 6.74 - 6.79 (1H, m), 6.86 - 6.89 (1H, m), 6.91 - 6.96 (1H, m), 7.13 - 7.18 (1H, m), 7.20 - 7.25 (1H, m), 7.29 - 7.31 (1H, m) |

(continued)

| Hydrolysis methods: | | | | | | |
|---|---|---|---|---|---|---|
| A, LiOH / aq THF or aq MeOH or aq THF - MeOH | | | | | | |
| B, KOTMS / THF / RT 3 days | | | | | | |
| C, TFA/ dichloromethane | | | | | | |
| D, 6M HCl aq reflux | | | | | | |
| E, 4M HCl / dioxan | | | | | | |
| Example | Name | Hydrolysis Method | MS $(M+H)^+$ ES +ve | retention time | HPLC system | $^1H$ NMR $\delta_{(CD3OD+1drop\ N\alpha O\Delta)}$ |
| 73 | 2-(4-{[4-(4-chloro-3-methylphenoxy) piperidin-1-yl]methyl} piperidin-1-yl)-5-methylbenzoic acid | A | 455 / 457 -ve | | | 1.38 - 1.52 (2H, m), 1.56 - 1.69 (1H, m), 1.72 - 1.84 (4H, m), 1.96 - 2.06 (2H, m), 2.23 - 2.38 (10H, m), 2.61 - 2.80 (4H, m), 3.44 (2H, d), 4.30 - 4.40 (1H, m), 6.76 (1H, dd), 6.86 - 6.89 (2H, m), 7.00 (1H, dd), 7.16 (1H, d), 7.22 (1H, d) |
| 74 | 4-chloro-2-(4-{[4-(4-chloro-3-methylphenoxy) piperidin-1-yl]methyl} piperidin-1-yl)benzoic acid | A | 475 / 477 -ve | | | 1.37 - 1.54 (2H, m), 1.61 - 1.85 (5H, m), 1.96 - 2.08 (2H, m), 2.26 - 2.39 (7H, m), 2.64 - 2.82 (4H, m), 3.46 - 3.54 (2H, m), 4.32 - 4.41 (1H, m), 6.73 - 6.79 (1H, m), 6.82 - 6.91 (3H, m), 7.20 - 7.25 (1H, m), 7.31-7.34 (1H, m) |
| 75 | 3-chloro-2-(4-{[4-(4-chloro-3-methylphenoxy) piperidin- 1-yl]methyl} piperidin-1-yl)benzoic acid | A | 475 / 477 -ve | | | 1.33 - 1.49 (2H, m), 1.63 - 1.85 (5H, m), 1.96 - 2.07 (2H, m), 2.25 - 2.39 (7H, m), 2.71 - 2.82 (2H, m), 3.18 - 3.26 (4H, m), 4.32 - 4.42 (1H, m), 6.74 - 6.79 (1H, m), 6.88 (1H, d), 6.95 (1H, t), 7.18 - 7.30 (3H, m) |

(continued)

| Hydrolysis methods: | | | | | | |
|---|---|---|---|---|---|---|
| A, LiOH / aq THF or aq MeOH or aq THF - MeOH | | | | | | |
| B, KOTMS / THF / RT 3 days | | | | | | |
| C, TFA/ dichloromethane | | | | | | |
| D, 6M HCl aq reflux | | | | | | |
| E, 4M HCl / dioxan | | | | | | |
| Example | Name | Hydrolysis Method | MS $(M+H)^+$ ES +ve | retention time | HPLC system | $^1H$ NMR $\delta_{(CD3OD+1drop\ N\alpha O\Delta)}$ |
| 76 | 2-(4-{[4-(4-chloro-3-methylphenoxy) piperidin-1- yl]methyl} piperidin-1-yl)-3-methoxybenzoic acid | A | 473 / 475 | | | 1.27 - 1.45 (2H, m), 1.59 - 1.84 (5H, m), 1.95 - 2.07 (2H, m), 2.23 - 2.39 (7H, m), 2.67 - 2.81 (2H, m), 3.16 - 3.22 (4H, m), 3.82 (3H, s), 4.31 - 4.42 (1H, m), 6.73 - 6.89 (4H, m), 6.96 - 7.03 (1H, m), 7.19 - 7.25 (1H, m) |
| 77 | 2-(4-{[4-(3-chloro-4-cyanophenoxy) piperidin-1- yl]methyl} piperidin-1-yl)-6-fluorobenzoic acid | A | 470 / 472 -ve | | | 1.35 - 1.50 (2H, m), 1.58 - 1.72 (1H, m), 1.76 - 1.89 (4H, m), 2.00 - 2.11 (2H, m), 2.29 (2H, d), 2.38 (2H, t), 2.65 - 2.80 (4H, m), 3.52 - 3.59 (2H, m), 4.53 - 4.63 (1H, m), 6.67 (1H, t), 6.80 (1H, d), 7.04 - 7.18 (2H, m), 7.21 (1H, d), 7.71 (1H, d) |
| 78 | 2-(4-{[4-(3-chloro-4-cyanophenoxy) piperidin-1-yl]methyl} piperidin-1-yl)-4-methoxybenzoic acid | A | 482/484 -ve | | | 1.38 - 1.53 (2H, m), 1.58 - 1.72 (1H, m), 1.76 - 1.89 (4H, m), 2.01 - 2.11 (2H, m), 2.31 (2H, d), 2.33 - 2.43 (2H, m), 2.61 - 2.81 (4H, m), 3.46 - 3.54 (2H, m), 3.78 (3H, s), 4.53 - 4.62 (1H, m), 6.41 - 6.48 (2H, m), 7.06 (1H, dd), 7.20 (1H, d), 7.39 (1H, d), 7.72 (1H, d) |

(continued)

| Hydrolysis methods: | | | | | | |
|---|---|---|---|---|---|---|
| A, LiOH / aq THF or aq MeOH or aq THF - MeOH | | | | | | |
| B, KOTMS / THF / RT 3 days | | | | | | |
| C, TFA/ dichloromethane | | | | | | |
| D, 6M HCl aq reflux | | | | | | |
| E, 4M HCl / dioxan | | | | | | |
| Example | Name | Hydrolysis Method | MS $(M+H)^+$ ES +ve | retention time | HPLC system | $^1H$ NMR $\delta_{(CD3OD+1drop\ N\alpha O\Delta)}$ |
| 79 | 2-(4-{[4-(3,4-dichlorobenzyl) piperidin-1-yl]methyl} piperidin-1-yl)-5-methylbenzoic acid | A | 475 / 477 | | | 1.23 (4H, m), 1.48 - 1.65 (4H, m), 1.70 - 1.81 (2H, m), 1.85 - 1.98 (2H, m), 2.24 (5H, d), 2.55 (2H, d), 2.58 - 2.70 (2H, m), 2.89 - 2.97 (2H, m), 3.38 - 3.47 (2H, m), 6.86 (1H, d), 6.96 - 7.01 (1H, m), 7.09 - 7.17 (2H, m), 7.35 (1H, d), 7.42 (1H, d) |
| 80 | 2-(4-{[4-(4-chlorobenzyl)piperidin-1- yl]methyl}piperidin-1-yl)-5- methylbenzoic acid | A | 441 / 443 | | | 1.26 - 1.67 (8H, m), 1.72 - 1.82 (2H, m), 1.86 - 1.97 (2H, m), 2.21 - 2.28 (5H, m), 2.55 (2H, d), 2.59 - 2.69 (2H, m), 2.88 - 2.97 (2H, m), 3.39 - 3.47 (2H, m), 6.86 (1H, d), 6.96 - 7.01 (1H, m), 7.13 - 7.19 (3H, m), 7.24 - 7.27 (1H, m), 7.27 - 7.30 (1H, m) |
| 81 | 2-(4-{[4-(4-chlorobenzyl)piperidin-1-yl]methyl}piperidin-1-yl)-3-methoxybenzoic acid | B | 457 / 459 | | | 1.16 - 1.34 (4H, m), 1.44 - 1.62 (6H, m), 2.10 (2H, d), 2.43 (2H, d), 2.75 - 2.86 (2H, m), 3.01 - 3.14 (4H, m), 3.70 (3H, s), 6.68 - 6.77 (2H, m), 6.87 (1H, t), 7.01 - 7.09 (2H, m), 7.12 - 7.20 (2H, m) |

(continued)

| Hydrolysis methods: | | | | | | |
|---|---|---|---|---|---|---|
| A, LiOH / aq THF or aq MeOH or aq THF - MeOH | | | | | | |
| B, KOTMS / THF / RT 3 days | | | | | | |
| C, TFA/ dichloromethane | | | | | | |
| D, 6M HCl aq reflux | | | | | | |
| E, 4M HCl / dioxan | | | | | | |
| Example | Name | Hydrolysis Method | MS $(M+H)^+$ ES +ve | retention time | HPLC system | $^1H$ NMR $\delta_{(CD3OD+1drop\ N\alpha O\Delta)}$ |
| 82 | 2-(4-{[4-(3,4-dichlorobenzyl) piperidin-1-yl]methyl} piperidin-1-yl)-3-methoxybenzoic acid | B | 491 / 493 | 1.94 | Standard | 1.15 - 1.33 (4H, m), 1.44 - 1.62 (6H, m), 1.73 - 1.86 (2H, m), 2.11 (2H, d), 2.44 (2H, d), 2.73 - 2.88 (2H, m), 3.00 - 3.10 (4H, m), 3.69 (3H, s), 6.67 - 6.75 (2H, m), 6.82 - 6.90 (1H, m), 6.97 - 7.03 (1H, m), 7.22 - 7.25 (1H, m), 7.28 -7.33 (1H, m) |
| 83 | 2-(4-{[4-(4-fluorobenzyl)piperidin-1- yl]methyl}piperidin-1-yl)-5- ethylbenzoic acid | B | 425 / 427 | 2.33 | Standard | 1.24 - 1.63 (8H, m), 1.70 - 1.77 (2H, m), 1.83 - 1.92 (2H, m), 2.18 - 2.23 (5H, m), 2.51 (2H, d), 2.57 - 2.65 (2H, m), 2.86 - 2.93 (2H, m), 3.36 - 3.43 (2H, m), 6.83 (1H, d), 6.93 - 6.99 (3H, m), 7.10 - 7.16 (3H, m) |
| 84 | 2-(4-{[4-(4-fluorobenzyl)piperidin-1-yl]methyl}piperidin-1-yl)-3-methoxybenzoic acid | B | | | | 1.26 - 1.40 (5H, m), 1.55 - 1.69 (5H, m), 1.83 - 1.91 (2H, m), 2.19 (2H, d), 2.51 (2H, d), 2.86 - 2.93 (2H, m), 3.10 - 3.18 (4H, m), 3.78 (3H, s), 6.78 - 6.82 (2H, m), 6.93 - 6.99 (3H, m), 7.11 - 7.16 (2H, m) |

(continued)

| Hydrolysis methods: | | | | | | |
|---|---|---|---|---|---|---|
| A, LiOH / aq THF or aq MeOH or aq THF - MeOH | | | | | | |
| B, KOTMS / THF / RT 3 days | | | | | | |
| C, TFA/ dichloromethane | | | | | | |
| D, 6M HCl aq reflux | | | | | | |
| E, 4M HCl / dioxan | | | | | | |
| Example | Name | Hydrolysis Method | MS $(M+H)^+$ ES +ve | retention time | HPLC system | $^1H$ NMR δ(CD3OD+1drop NαOΔ) |
| 85 | 2-(4-{[3-(3,4-dichlorophenoxy) azetidin-1- yl]methyl} piperidin-1-yl)-5-fluorobenzoic acid | A | 451 / 453 -ve | | | 1.39 - 1.53 (3H, m), 1.68 - 1.78 (2H, m), 2.49 - 2.53 (2H, m), 2.58 - 2.69 (2H, m), 3.17 - 3.24 (2H, m), 3.36 - 3.43 (2H, m), 3.80 - 3.87 (2H, m), 4.80 - 4.86 (1H, m), 6.80 (1H, dd), 6.87 - 7.05 (4H, m), 7.42 (1H, d) |
| 86 | 2-(4-{[3-(3,4-dichlorophenoxy) azetidin-1-yl]methyl} piperidin-1-yl)benzoic acid | A | 433 / 435 -ve | | | 1.38 - 1.56 (3H, m), 1.69 - 1.78 (2H, m), 2.51 (2H, s), 2.67 (2H, t), 3.17 - 3.24 (2H, m), 3.45 - 3.53 (2H, m), 3.79 - 3.87 (2H, m), 4.78 - 4.87 (1H, m), 6.77 - 6.97 (3H, m), 6.99 - 7.02 (1H, m), 7.12 - 7.21 (1H, m), 7.33 (1H, d), 7.41 (1H, d) |
| 87 | 2-(4-{[(3S)-3-(4-chlorophenoxy) pyrrolidin-1-yl]methyl} piperidin-1-yl)benzoic acid | A | 413 / 415 -ve | | | 1.40 - 1.55 (2H, m), 1.55 - 1.66 (1H, m), 1.78 - 1.87 (2H, m), 1.93 - 1.99 (1H, m), 2.27 - 2.85 (8H, m), 2.91 - 2.98 (1H, m), 3.46 - 3.54 (2H, m), 4.84 - 4.91 (1H, m), 6.84 - 6.91 (3H, m), 6.97 (1H, d), 7.15 - 7.22 (1H, m), 7.26 (2H, dd), 7.34 (1H, dd) |

(continued)

| Hydrolysis methods: | | | | | | |
|---|---|---|---|---|---|---|
| A, LiOH / aq THF or aq MeOH or aq THF - MeOH | | | | | | |
| B, KOTMS / THF / RT 3 days | | | | | | |
| C, TFA/ dichloromethane | | | | | | |
| D, 6M HCl aq reflux | | | | | | |
| E, 4M HCl / dioxan | | | | | | |
| Example | Name | Hydrolysis Method | MS $(M+H)^+$ ES +ve | retention time | HPLC system | $^1H$ NMR $\delta_{(CD3OD+1drop\ N\alpha O\Delta)}$ |
| 88 | 2-(4-{[(3S)-3-(4-chlorophenoxy) pyrrolidin-1-yl]methyl} piperidin-1-yl)-5-fluorobenzoic acid | A | 431 / 433 -ve | | | 1.39 - 1.53 (2H, m), 1.55 - 1.65 (1H, m), 1.77 - 1.86 (2H, m), 1.88 - 1.99 (1H, m), 2.27 - 2.50 (3H, m), 2.53 - 2.72 (3H, m), 2.74 - 2.85 (2H, m), 2.90 - 2.97 (1H, m), 3.37 - 3.44 (2H, m), 4.84 - 4.92 (1H, m), 6.86 - 7.05 (5H, m), 7.26 (2H, dd) |
| 89 | 2-(4-{[3-(3-chloro-4-cyanophenoxy) azetidin-1-yl]methyl} piperidin-1-yl)benzoic acid | A | 424 / 426 -ve | | | 1.38 - 1.58 (3H, m), 1.69 - 1.81 (2H, m), 2.49 - 2.56 (2H, m), 2.61 - 2.74 (2H, m), 3.21 - 3.30 (2H, m), 3.44 - 3.54 (2H, m), 3.81 - 3.91 (2H, m), 4.91 - 5.00 (1H, m), 6.84 - 7.00 (3H, m), 7.10 - 7.23 (2H, m), 7.31 - 7.38 (1H, m), 7.71 - 7.78 (1H, m) |
| 90 | 2-(4-{[4-(3-chloro-4-cyano-2-methylphenoxy) piperidin-1-yl]methyl} piperidin-1-yl)benzoic acid | A | 466/468 -ve | | | 1.39 - 1.54 (2H, m), 1.59 - 1.74 (1H, m), 1.77 - 1.93 (4H, m), 2.03 - 2.13 (2H, m), 2.29 - 2.35 (5H, m), 2.39 - 2.49 (2H, m), 2.64 - 2.76 (4H, m), 3.47 - 3.55 (2H, m), 4.59 - 4.67 (1H, m), 6.88 (1H, t), 6.97 (1 H, d), 7.10 (1H, d), 7.20 (1H, dd), 7.35 (1H, dd), 7.63 (1H, d) |

(continued)

| Hydrolysis methods: | | | | | | |
|---|---|---|---|---|---|---|
| A, LiOH / aq THF or aq MeOH or aq THF - MeOH | | | | | | |
| B, KOTMS / THF / RT 3 days | | | | | | |
| C, TFA/ dichloromethane | | | | | | |
| D, 6M HCl aq reflux | | | | | | |
| E, 4M HCl / dioxan | | | | | | |
| Example | Name | Hydrolysis Method | MS $(M+H)^+$ ES +ve | retention time | HPLC system | $^1$H NMR $\delta_{(CD3OD+1drop\ N\alpha O\Delta)}$ |
| 91 | 2-(4-{[3-(3-chloro-4-cyanophenoxy) azetidin-1-yl]methyl} piperidin-1-yl)-5-fluorobenzoic acid | A | 442/444 -ve | | | 1.12 - 1.27 (2H, m), 1.71 - 1.89 (4H, m), 1.99 - 2.10 (2H, m), 2.25 (2H, d), 2.31 - 2.41 (3H, m), 2.63 - 2.78 (4H, m), 3.13 (2H, d), 4.51 - 4.62 (1H, m), 7.05 (1H, dd), 7.21 (1H, d), 7.70 (1H, d) |
| 92 | 2-(4-{[4-(3,4-dichlorophenoxy) piperidin-1-yl]methyl} piperidin-1-yl)nicotinic acid | D | 462/464 -ve | 1.36 | Standard | 1.20 - 1.32 (3H, m), 1.65 - 1.76 (4H, m), 1.93 - 2.01 (2H, m), 2.20 (2H, d), 2.22 - 2.30 (2H, m), 2.65 - 2.73 (2H, m), 2.81 (2H, t), 4.00 (2H, d), 4.37 - 4.44 (1H, m), 6.64 (1H, dd), 6.95 (1H, dd), 7.16 (1H, d), 7.44 (1H, d), 7.57 (1H, dd), 7.99 (1H, dd) |
| 93 | 2-[4-({4-[4-(aminocarbonyl)-3-chlorophenoxy] piperidin-1-yl}methyl) piperidin-1-yl]-6-fluorobenzoic acid | A | 488 / 490 -ve | | | 1.35 - 1.50 (2H, m), 1.58 - 1.72 (1H, m), 1.76 - 1.87 (4H, m), 1.99 - 2.10 (2H, m), 2.27 - 2.41 (4H, m), 2.66 - 2.80 (4H, m), 3.51 - 3.60 (2H, m), 4.44 - 4.54 (1H, m), 6.67 (1H, t), 6.81 (1H, d), 6.96 (1H, dd), 7.03 (1H, d), 7.14 (1H, td), 7.52 (1H, d) |

(continued)

| Hydrolysis methods: | | | | | | |
|---|---|---|---|---|---|---|
| A, LiOH / aq THF or aq MeOH or aq THF - MeOH | | | | | | |
| B, KOTMS / THF / RT 3 days | | | | | | |
| C, TFA/ dichloromethane | | | | | | |
| D, 6M HCl aq reflux | | | | | | |
| E, 4M HCl / dioxan | | | | | | |
| Example | Name | Hydrolysis Method | MS $(M+H)^+$ ES +ve | retention time | HPLC system | $^1$H NMR $\delta_{(CD3OD+1drop\ N\alpha O\Delta)}$ |
| 94 | 3-(4-{[4-(3,4-dichlorophenoxy) piperidin-1-yl]methyl} piperidin-1-yl)benzoic acid | A | 463 / 467 (M+H) | | | 1.36 - 1.45 (2H, m), 1.73 - 1.86 (3H, m), 1.91 - 1.95 (2H, m), 2.03 - 2.08 (2H, m), 2.31 - 2.33 (2H, d), 2.36 - 2.40 (2H, t), 2.72 - 2.77 (4H, m), 3.73 - 3.76 (2H, d), 4.38 - 4.48 (1H, m), 6.91 - 6.94 (1H, dd), 7.08 - 7.12 (2H, m), 7.24 - 7.28 (1H, t), 7.40 - 7.43 (1H, d), 7.47 - 7.49 (1H, d), 7.67 (1H, s). |
| 95 | 3-(4-{[4-(4-chlorophenoxy) piperidin-1- yl]methyl} piperidin-1- yl)benzoic acid | A Isolated as impurity in example 95 | 429 / 431 (M+H) | | | $\delta_{(CDCL3)}$ 1.44 (2H, q), 1.81 - 2.10 (5H, m), 2.26 - 2.38 (2H, m), 2.59 (2H, d), 2.74 (2H, t), 2.84 - 3.02 (4H, m), 3.73 (2H, d), 4.52 (1H, s), 6.83 (2H, d), 7.00 - 7.07 (1H, m), 7.16 - 7.29 (3H, m), 7.50 (1H, d), 7.62 (1H, s) |
| 96 | 3-(4-{[4-(3,4-dichloro-2-methylphenoxy) piperidin-1-yl]methyl} piperidin-1-yl)benzoic acid | A | 477 / 481 (M+H) | | | 1.29 - 1.49 (2H, m), 1.70 - 1.98 (5H, m), 2.01 - 2.12 (2H, m), 2.32 (2H, d), 2.35 (3H, s), 2.38 - 2.48 (2H, m), 2.74 (4H, t), 3.74 (2H, d), 4.43 - 4.53 (1 H, m), 6.96 (1H, d), 7.09 (1H, d), 7.29 (2H, mult), 7.49 (1 H, d), 7.67 (1H, s) |

(continued)

| Hydrolysis methods: | | | | | | |
|---|---|---|---|---|---|---|
| A, LiOH / aq THF or aq MeOH or aq THF - MeOH | | | | | | |
| B, KOTMS / THF / RT 3 days | | | | | | |
| C, TFA/ dichloromethane | | | | | | |
| D, 6M HCl aq reflux | | | | | | |
| E, 4M HCl / dioxan | | | | | | |
| Example | Name | Hydrolysis Method | MS $(M+H)^+$ ES +ve | retention time | HPLC system | $^1$H NMR δ(CD3OD+1drop NαOΔ) |
| 97 | 3-(4-{(4-(3,4-dichlorophenoxy) piperidin-1-yl]methyl} piperidin-1-yl)- 4-methylbenzoic acid | C | 477 / 479 | | | 1.18 - 1.37 (3H, m), 1.56 - 1.74 (3H, m), 1.74 - 1.82 (2H, m), 1.88 - 1.97 (2H, m), 2.20 (3H, s), 2.21 - 2.30 (3H, m), 2.54 - 2.70 (4H, m), 2.99 - 3.06 (2H, m), 4.26 - 4.35 (1H, m), 6.80 (1H, dd), 7.00 (1H, d), 7.03 (1H, d), 7.28 (1H, d), 7.44 (1H, dd), 7.59 (1H, d) |
| 98 | 3-(4-{[4-(3,4-dichlorophenoxy) piperidin-1-yl]methyl} piperidin-1-yl)- 2-methylbenzoic acid | C | 477 / 479 | | | 1.26 - 1.46 (3H, m), 1.62 - 1.73 (1H, m), 1.73 - 1.89 (4H, m), 1.97 - 2.07 (2H, m), 2.27 - 2.33 (3H, m), 2.36 (3H, s), 2.62 (2H, t), 2.71 - 2.80 (2H, m), 3.05 - 3.12 (2H, m), 4.36 - 4.44 (1H, m), 6.89 (1H, dd), 6.97 (1H, dd), 7.02 - 7.08 (2H, m), 7.10 (1H, d), 7.38 (1H, d) |
| 99 | 3-(4-{[4-(3,4-dichlorophenoxy) piperidin-1-yl]methyl} piperidin-1-yl)- 5-methylbenzoic acid | E | 477 / 479 | 0.49 | fast | 1.17 - 1.33 (3H, m), 1.53 - 1.73 (3H, m), 1.75 - 1.82 (2H, m), 1.87 - 1.96 (2H, m), 2.15 - 2.28 (6H, m), 2.55 - 2.69 (4H, m), 3.55 - 3.62 (2H, m), 4.25 - 4.34 (1H, m), 6.77 - 6.81 (2H, m), 7.00 (1H, d), 7.18 - 7.20 (1H, m), 7.28 (1H, d), 7.33 (1H, s) |

(continued)

| Hydrolysis methods: | | | | | | |
|---|---|---|---|---|---|---|
| A, LiOH / aq THF or aq MeOH or aq THF - MeOH | | | | | | |
| B, KOTMS / THF / RT 3 days | | | | | | |
| C, TFA/ dichloromethane | | | | | | |
| D, 6M HCl aq reflux | | | | | | |
| E, 4M HCl / dioxan | | | | | | |
| Example | Name | Hydrolysis Method | MS $(M+H)^+$ ES +ve | retention time | HPLC system | $^1$H NMR $\delta_{(CD3OD+1drop\ N\alpha O\Delta)}$ |
| 100 | 4-chloro-3-(4-{[4-(3,4-dichlorophenoxy) piperidin-1-yl]methyl} piperidin-1- yl)benzoic acid | E | 498 / 500 | | | 1.37 - 1.49 (2H, m), 1.65 - 1.83 (3H, m), 1.84 - 1.91 (2H, m), 1.97 - 2.06 (2H, m), 2.28 - 2.39 (4H, m), 2.64 - 2.79 (4H, m), 3.33 - 3.41 (2H, m), 4.36 - 4.44 (1 H, m), 6.89 (1H, dd), 7.08 (1H, d), 7.31 (1H, d), 7.38 (1H, d), 7.55 (1H, dd), 7.74 (1H, d) |
| 101 | 3-(4-{[4-(3,4-dichlorophenoxy) piperidin-1-yl]methyl} piperidin-1-yl)-4-methoxybenzoic acid | E | 493 / 495 | 1.46 | Standard | 1.38 - 1.50 (2H, m), 1.64 - 1.83 (3H, m), 1.83 -1.90 (2H, m), 1.97 - 2.06 (2H, m), 2.27 - 2.38 (4H, m), 2.57 - 2.66 (2H, m), 2.71 - 2.80 (2H, m), 3.40 - 3.46 (2H, m), 3.89 (3H, s), 4.36 - 4.43 (1H, m), 6.87 - 6.91 (1H, m), 6.92 (1H, d), 7.10 (1H, d), 7.37 (1H, d), 7.64 - 7.70 (2H, m) |
| 102 | 3-(4-{[4-(2,4-dichloro-3-methylphenoxy) piperidin-1-yl]methyl} piperidin-1-yl)benzoic acid | A | | | | 1.27 - 1.44 (2H, m), 1.67 - 1.77 (1H, m), 1.80 - 1.94 (4H, m), 1.96 - 2.06 (2H, m), 2.29 (2H, d), 2.35 - 2.43 (2H, m), 2.45 (3H, s), 2.65 - 2.80 (4H, m), 3.71 (2H, d), 4.42 - 4.51 (1H, m), 6.95 (1H, d), 7.05 (1H, dd), 7.19 - 7.27 (2H, m), 7.44 (1H, d), 7.63 (1H, t) |

(continued)

| | | | | | | |
|---|---|---|---|---|---|---|
| Hydrolysis methods: | | | | | | |
| A, LiOH / aq THF or aq MeOH or aq THF - MeOH | | | | | | |
| B, KOTMS / THF / RT 3 days | | | | | | |
| C, TFA/ dichloromethane | | | | | | |
| D, 6M HCl aq reflux | | | | | | |
| E, 4M HCl / dioxan | | | | | | |
| Example | Name | Hydrolysis Method | MS $(M+H)^+$ ES +ve | retention time | HPLC system | $^1$H NMR δ(CD3OD+1drop NαOΔ) |
| 103 | 2-chloro-5-(4-{[4-(3,4-dichlorophenoxy) piperidin-1-yl]methyl} piperidin-1-yl)benzoic acid | E | 497 / 499 | 1.47 | Standard | 1.24 - 1.40 (2H, m), 1.60 - 1.82 (3H, m), 1.82 - 1.91 (2H, m), 1.96 - 2.05 (2H, m), 2.24 - 2.29 (2H, m), 2.29 - 2.38 (2H, m), 2.64 - 2.78 (4H, m), 3.62 - 3.69 (2H, m), 4.35 - 4.43 (1H, m), 6.87 (1H, dd), 6.89 (1H, dd), 7.04 (1H, d), 7.09 (1H, d), 7.15 (1H, d), 7.37 (1H, d) |
| 104 | 6-(4-{[4-(3,4-dichlorophenoxy) piperidin-1-yl]methyl} piperidin-1-yl)pyridine-2-carboxylic acid | E | 464 / 466 | 1.47 | Standard | 1.16 - 1.30 (2H, m), 1.72 - 1.90 (5H, m), 1.96 - 2.05 (2H, m), 2.23 - 2.29 (2H, m), 2.29 - 2.38 (2H, m), 2.70 - 2.86 (4H, m), 4.35 - 4.45 (3H, m), 6.80 (1H, d), 6.89 (1H, dd), 7.09 (1H, d), 7.25 (1H, d), 7.37 (1H, d), 7.54 (1H, dd) |
| 105 | 5-(4-{[4-(3,4-dichlorophenoxy) piperidin-1-yl]methyl} piperidin-1-yl)-2-methylnicotinic acid | E | 478 / 480 | 1.31 | Standard | 1.28 - 1.40 (2H, m), 1.68 - 1.83 (3H, m), 1.85 - 1.93 (2H, m), 1.97 - 2.05 (2H, m), 2.26 - 2.30 (2H, m), 2.30 - 2.38 (2H, m), 2.54 - 2.57 (3H, m), 2.69 - 2.79 (4H, m), 3.65 - 3.73 (2H, m), 4.35 - 4.43 (1 H, m), 6.89 (1 H, dd), 7.10 (1 H, d), 7.37 (1H, d), 7.49 (1H, d), 8.02 (1H, d) |

(continued)

| Hydrolysis methods: | | | | | | |
|---|---|---|---|---|---|---|
| A, LiOH / aq THF or aq MeOH or aq THF - MeOH | | | | | | |
| B, KOTMS / THF / RT 3 days | | | | | | |
| C, TFA/ dichloromethane | | | | | | |
| D, 6M HCl aq reflux | | | | | | |
| E, 4M HCl / dioxan | | | | | | |
| Example | Name | Hydrolysis Method | MS $(M+H)^+$ ES +ve | retention time | HPLC system | $^1H$ NMR $\delta_{(CD3OD+1drop\ N\alpha O\Delta)}$ |
| 106 | 3-(4-{[4-(3,4-dichlorobenzyl) piperidin-1-yl]methyl} piperidin-1-yl)benzoic acid | D | | | | 1.15 - 1.32 (4H, m), 1.40 - 1.66 (4H, m), 1.73 - 1.86 (4H, m), 2.13 (2H, d), 2.44 (2H, d), 2.55 - 2.64 (2H, m), 2.79 - 2.86 (2H, m), 3.56 - 3.63 (2H, m), 6.91 - 6.97 (1H, m), 7.00 (1H, dd), 7.11 (1H, t), 7.24 (1H, d), 7.28 - 7.36 (2H, m), 7.52 (1H, s) |

EXAMPLE 107

Pharmacological Analysis: Calcium flux $[Ca^{2+}]_i$ assay

Human eosinophils

Human eosinophils were isolated from EDTA anticoagulated peripheral blood as previously described (Hansel et al., J. Immunol. Methods, 1991, 145, 105-110). The cells were resuspended ($5x10^6$ $ml^{-1}$) and loaded with 5μM FLUO-3/AM + Pluronic F127 2.2μl/ml (Molecular Probes) in low potassium solution (LKS; NaCl 118mM, $MgSO_4$ 0.8mM, glucose 5.5mM, $Na_2CO_3$ 8.5mM, KCl 5mM, HEPES 20mM, $CaCl_2$ 1.8mM, BSA 0.1%, pH 7.4) for one hour at room temperature. After loading, cells were centrifuged at 200g for 5min and resuspended in LKS at $2.5x10^6$ $ml^{-1}$. The cells were then transferred to 96 well FLIPr plates (Poly-D-Lysine plates from Becton Dickinson pre-incubated with 5μM fibronectin for two hours) at 25μl/well. The plate was centrifuged at 200g for 5min and the cells were washed twice with LKS (200μl; room temperature).

A compound of the Examples was pre-dissolved in DMSO and added to a final concentration of 0.1%(v/v) DMSO. Assays were initiated by the addition of an $A_{50}$ concentration of eotaxin and the transient increase in fluo-3 fluorescence ($1_{Ex}$ =490nm and $1_{Em}$ = 520nm) monitored using a FLIPR (Fluorometric Imaging Plate Reader, Molecular Devices, Sunnyvale, U.S.A.).

Compounds of the Examples were found to be antagonists if the increase in fluorescence induced by eotaxin (a selective CCR3 agonist) was inhibited in a concentration dependent manner. The concentration of antagonist required to inhibit the fluorescence by 50% can be used to determine the $IC_{50}$ for the antagonist at the CCR3 receptor.

EXAMPLE 108

Human eosinophil chemotaxis

Human eosinophils were isolated from EDTA anticoagulated peripheral blood as previously described (Hansel et al., J. Immunol. Methods, 1991, 145, 105-110). The cells were resuspended at $10x10^6$ $ml^{-1}$ in RPMI containing 200 IU/ml penicillin, 200 μg/ml streptomycin sulfate and supplemented with 10% HIFCS, at room temperature.

Eosinophils (700 μl) were pre-incubated for 15 mins at 37° C with 7 μl of either vehicle or compound (100x

required final concentration in 10% DMSO). The chemotaxis plate (ChemoTx, 3μm pore, Neuroprobe) was loaded by adding 28μl of a concentration of eotaxin 0.1 to 100nM (a selective CCR3 agonist over this concentration range) containing a concentration of a compound according to the Examples or solvent to the lower wells of the chemotaxis plate. The filter was then placed over the wells and 25 μl of eosinophil suspension were added to the top of the filter. The plate was incubated for 1 hr at 37° C in a humidified incubator with a 95% air/5% $CO_2$ atmosphere to allow chemotaxis.

The medium, containing cells that had not migrated, was carefully aspirated from above the filter and discarded. The filter was washed once with phosphate buffered saline (PBS) containing 5 mM EDTA to remove any adherent cells. Cells that had migrated through the filter were pelleted by centrifugation (300xg for 5 mins at room temperature) and the filter removed and the supernatant transferred to each well of a 96-well plate (Costar). The pelleted cells were lysed by the addition of 28 μl of PBS containing 0.5% Triton x100 followed by two cycles of freeze/thawing. The cell lysate was then added to the supernatant. The number of eosinophils migrating was quantified according to the method of Strath et al., J. Immunol. Methods, 1985, 83, 209 by measuring eosinophil peroxidase activity in the supernatant.

Compounds of the Examples were found to be antagonists of eotaxin mediated human eosinophil chemotaxis if the concentration response to eotaxin was shifted to the right of the control curve. Measuring the concentration of eotaxin required to give 50% chemotaxis in the presence or absence of compounds enables the apparent affinity of the compounds at CCR3 to be calculated.

EXAMPLE 109

Guinea-pig isolated trachea

(See for example, Harrison, R.W.S., Carswell, H. & Young, J.M. (1984) European J. Pharmacol., 106,405-409.)

Male albino Dunkin-Hartley guinea-pigs (250g) were killed by cervical dislocation and the whole trachea removed. After clearing the adherent connective tissue, the trachea was cut into six ring segments each three cartilage bands wide and then suspended in 20ml organ baths containing Krebs-Henseleit solution of the following composition (mM): NaCl 117.6, $NaH_2PO_4$ 0.9, $NaHCO_3$ 25.0, $MgSO_4$ 1.2, KCl 5.4, $CaCl_2$ 2.6 and glucose 11.1. The buffer was maintained at 37°C and gassed with 5% $CO_2$ in oxygen. Indomethacin (2.8μM) was added to the Krebs solution to prevent development of smooth muscle tone due to the synthesis of cyclo-oxygenase products. The tracheal rings were suspended between two parallel tungsten wire hooks, one attached to an Ormed beam isometric force transducer and the other to a stationary support in the organ bath. Changes in isometric force were recorded on 2-channel Sekonic flat bed chart recorders.

Experimental protocols

At the beginning of each experiment a force of 1g was applied to the tissues and this was reinstated over a 60 minute equilibration period until a steady resting tone was achieved. Subsequently, a cumulative histamine concentration effect (E/[A]) curve was constructed at 0.5 $\log_{10}$ unit increments, in each tissue. The tissues were then washed and approximately 30 minutes later, test compound or vehicle (20% DMSO) was added. Following an incubation period of 60 minutes a second E/[A] curve was performed to histamine.

Contraction responses were recorded as a percentage of the first curve maximum. Data analysis

Experimental E/[A] curve data were analysed for the purposes of estimating the potencies (p[$A_{50}$) values) of histamine in the absence and presence of the test compound. Affinity ($pA_2$) values of test compounds were subsequently calculated using the following equation:

$$\log(r-1) = \log[B] + pA_2$$

where r = $[A]_{50}$ in presence of test compound/$[A]_{50}$ in absence of antagonist and [B] is the concentration of test compound. Compounds of the Examples were found to be H1 antagonists.

EXAMPLE 110

Histamine H1 receptor binding activity of compounds of the invention was assessed by competition displacement of 1nM [3H]-pyrilamine (Amersham, Bucks, Product code TRK 608, specific activity 30Ci/mmol) to 2μg membranes prepared from recombinant CHO-K1 cells expressing the human H1 receptor (Euroscreen SA, Brussels, Belgium, product code ES-390-M) in assay buffer (50mM Tris pH 7.4 containing 2mM $MgCl_2$, 250mM sucrose and 100mM NaCl) for 1 hour at room temperature.

The following compounds of the invention gave inhibition of [3H] pyrilimine binding:

| Example | H1 pKi |
|---|---|
| 1 | 8.6 |
| 32 | 8.0 |
| 33 | 6.6 |
| 34 | 6.2 |
| 36 | 8.0 |
| 38 | 7.2 |
| 47 | 8.3 |
| 48 | 7.1 |
| 49 | 7.3 |
| 55 | 6.5 |
| 59 | 8.8 |
| 63 | 7.3 |
| 68 | 7.8 |
| 74 | 8.6 |
| 96 | 7.7 |
| 99 | 8.1 |
| 106 | 7.7 |

## Claims

**1.** A compound of formula (I):

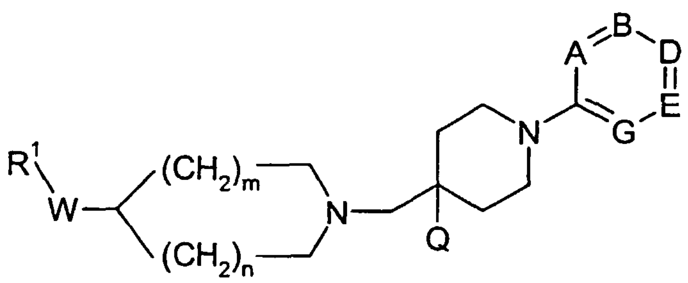

(I)

wherein:

n and m are, independently, 0 or 1;
A, B, D, E, G represent one each of $CCO_2R^5$, $CR^2$, $CR^3$, $CR^4$, and CH or N,
Q is hydrogen or hydroxy;
W is $CH_2$, O, NH or $N(C_{1-4}$ alkyl);
$R^1$ is phenyl optionally substituted by halogen, cyano, $C_{1-4}$ alkyl, $C_{1-4}$ haloalkyl, $C_1$-$_4$ alkoxy or $C_{1-4}$ haloalkoxy;
$R^2$, $R^3$ and $R^4$ are, independently, hydrogen, halogen, cyano, nitro, hydroxy, $NR^6R^7$, $C_{1-6}$ alkyl (optionally substituted with halogen), $C_{1-6}$ alkoxy (optionally substituted with halogen), $S(O)_p(C_{1-6}$ alkyl), $S(O)_qCF_3$ or $S(O)_2NR^7R^8$;
$R^5$ is hydrogen, $C_{1-6}$ alkyl or benzyl;
p and q are, independently, 0, 1 or 2;
$R^6$, $R^7$, $R^8$ and $R^9$ are, independently, hydrogen, $C_{1-6}$ alkyl (optionally substituted by halogen, hydroxy or $C_{3-6}$

cycloalkyl), $CH_2(C_{2-5}$ alkenyl), phenyl (itself optionally substituted by halogen, hydroxy, nitro, $NH_2$, $NH(C_{1-4}$ alkyl), $N(C_{1-4}$ alkyl$)_2$ (and these alkyl groups may join to form a ring as described for $R^6$ and $R^7$ below), $S(O)_2(C_{1-4}$ alkyl), $S(O)_2NH_2$, $S(O)_2NH(C_{1-4}$ alkyl), $S(O)_2N(C_{1-4}$ alkyl$)_2$ (and these alkyl groups may join to form a ring as described for $R^6$ and $R^7$ below), cyano, $C_{1-4}$ alkyl, $C_{1-4}$ alkoxy, $C(O)NH_2$, $C(O)NH(C_{1-4}$ alkyl), $C(O)N(C_{1-4}$ alkyl$)_2$ (and these alkyl groups may join to form a ring as described for $R^6$ and $R^7$ below), $CO_2H$, $CO_2(C_{1-4}$ alkyl), $NHC(O)(C_{1-4}$ alkyl), $NHS(O)_2(C_{1-4}$ alkyl), $C(O)(C_{1-4}$ alkyl), $CF_3$ or $OCF_3$) or heterocyclyl (itself optionally substituted by halogen, hydroxy, nitro, $NH_2$, $NH(C_{1-4}$ alkyl), $N(C_{1-4}$ alkyl$)_2$ (and these alkyl groups may join to form a ring as described for $R^6$ and $R^7$ below), $S(O)_2(C_{1-4}$ alkyl), $S(O)_2NH_2$, $S(O)_2NH(C_{1-4}$ alkyl), $S(O)_2N(C_{1-4}$ alkyl$)_2$ (and these alkyl groups may join to form a ring as described for $R^6$ and $R^7$ below), cyano, $C_{1-4}$ alkyl, $C_{1-4}$ alkoxy, $C(O)NH_2$, $C(O)NH(C_{1-4}$ alkyl), $C(O)N(C_{1-4}$ alkyl$)_2$ (and these alkyl groups may join to form a ring as described for $R^6$ and $R^7$ below), $CO_2H$, $CO_2(C_{1-4}$ alkyl), $NHC(O)(C_{1-4}$ alkyl), $NHS(O)_2(C_{1-4}$ alkyl), $C(O)(C_{1-4}$ alkyl), $CF_3$ or $OCF_3$);
alternatively $NR^6R^7$ or $NR^8R^9$ may, independently, form a 4-7 membered heterocyclic ring, azetidine, pyrrolidine, piperidine, azepine, morpholine or piperazine, the latter optionally substituted by $C_{1-4}$ alkyl on the distal nitrogen; or an N-oxide thereof; or a pharmaceutically acceptable salt thereof; or a solvate thereof.

**2.** A compound of formula (I) as claimed in claim 1 wherein W is O.

**3.** A compound as claimed in claim 1 or 2 wherein n and m are both 1.

**4.** A compound as claimed in claiin 1, 2 or 3 wherein $R^1$ is phenyl optionally substituted with halogen, cyano, $C_{1-4}$ alkyl or $C_{1-4}$ alkoxy.

**5.** A compound of formula (I) as claimed in any one of the preceding claims wherein Q is hydrogen.

**6.** A compound of formula (I) as claimed in any one of the preceding claims wherein one of A, B, D, E, G represent one each of $CCO_2R^5$, $CR^2$, $CR^3$, $CR^4$, and CH or N; wherein $R^2$, $R^3$ and $R^4$, are, independently, hydrogen, halogen, cyano, nitro, $C_{1-4}$ alkyl, $C_{1-4}$ alkoxy, $CF_3$, $OCF_3$, $S(O)_2(C_{1-4}$ alkyl) or $S(O)_2NH_2$; and $R^5$ is hydrogen or $C_{1-6}$ alkyl.

**7.** A process for preparing a compound of formula (I) as claimed in claim 1, the process comprising:

a. nucleophilic substitution of fluoride (II):

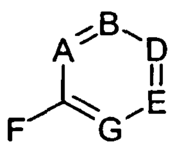

(II)

with a compound of formula (III):

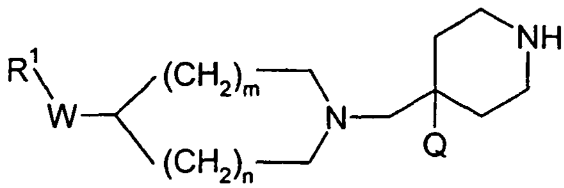

(III)

in the presence of a suitable solvent, at a suitable temperature, optionally in the presence of a suitable base;
b. when $R^5$ is hydrogen, said compound may be converted to a compound of the invention where $R^5$ is not hydrogen by a standard esterification method well known in the art;
c. when $R^5$ is not hydrogen said compound may be converted to a compound of the invention where $R^5$ is hydrogen by a standard ester hydrolysis method well known in the art; or
d. a Buchwald-Hartwig amination, where a compound of formula (VIII):

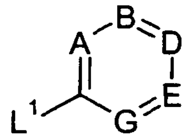

(VIII)

wherein $L^1$ is bromo or iodo; is reacted with a compound of formula (III):

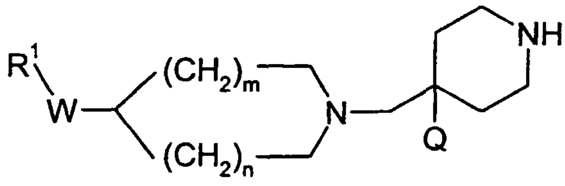

(III)

in the presence of a suitable palladium compound, a ligand, a base and a solvent, at a suitable temperature.

**8.** A pharmaceutical composition which comprises a compound of the formula (I), or a pharmaceutically acceptable salt thereof or solvate thereof as claimed in claim 1, and a pharmaceutically acceptable adjuvant, diluent or carrier.

**9.** A compound of the formula (I), or a pharmaceutically acceptable salt thereof or solvate thereof as claimed in claim 1, for use in therapy.

**10.** A compound of formula (I), or a pharmaceutically acceptable salt thereof or solvate thereof as claimed in claim 1, in the manufacture of a medicament for use in therapy.

## Patentansprüche

**1.** Verbindung der Formel (I):

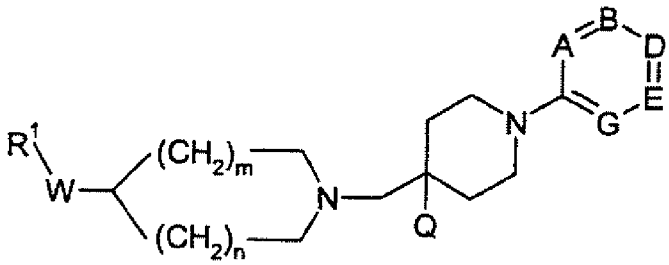

(I)

worin:

n und m unabhängig voneinander für 0 oder 1 stehen;
A, B, D, E und G für jeweils eine der Gruppen $CCO_2R^5$, $CR^2$, $CR^3$, $CR^4$ und CH oder N stehen;
Q für Wasserstoff oder Hydroxy steht;
W für $CH_2$, O, NH oder N($C_{1-4}$-Allkyl) steht;
$R^1$ für gegebenenfalls durch Halogen, Cyano, $C_{1-4}$-Alkyl, $C_{1-4}$-Halogenalkyl, $C_{1-4}$-Alkoxy oder $C_{1-4}$-Halogenalkoxy substituiertes Phenyl steht;
$R^2$, $R^3$ und $R^4$ unabhängig voneinander für Wasserstoff, Halogen, Cyano, Nitro, Hydroxy, $NR^6R^7$, $C_{1-6}$-Alkyl (gegebenenfalls substituiert durch Halogen), $C_{1-6}$-Alkoxy (gegebenenfalls substituiert durch Halogen), $S(O)_p$ ($C_{1-6}$-Alkyl), $S(O)_qCF_3$ oder $S(O)_2NR^7R^8$ stehen;
$R^5$ für Wasserstoff, $C_{1-6}$-Alkyl oder Benzyl steht;
p und q unabängig voneinander für 0, 1 oder 2 stehen;

$R^6$, $R^7$, $R^8$ und $R^9$ unabhängig voneinander für Wasserstoff, $C_{1-6}$-Alkyl (gegebenenfalls substituiert durch Halogen, Hydroxy oder $C_{3-6}$-Cycloalkyl), $CH_2(C_{2-5}$-Alkenyl), Phenyl (selbst gegebenenfalls substituiert durch Halogen, Hydroxy, Nitro, $NH_2$, $NH(C_{1-4}$-Alkyl), $N(C_{1-4}$-Alkyl$)_2$ (wobei diese Alkylgruppen zu einem Ring verbunden sein können, wie nachstehend für $R^6$ und $R^7$ beschrieben), $S(O)_2(C_{1-4}$-Alkyl), $S(O)_2NH_2$, $S(O)_2NH(C_{1-4}$-Alkyl), $S(O)_2N(C_{1-4}$-Alkyl$)_2$ (wobei diese Alkylgruppen zu einem Ring verbunden sein können, wie nachstehend für $R^6$ und $R^7$ beschrieben), Cyano, $C_{1-4}$-Alkyl, $C_{1-4}$-Alkoxy, $C(O)NH_2$, $C(O)NH(C_{1-4}$-Alkyl), $C(O)N(C_{1-4}$-Alkyl$)_2$ (wobei diese Alkylgruppen zu einem Ring verbunden sein können, wie nachstehend für $R^6$ und $R^7$ beschrieben), $CO_2H$, $CO_2(C_{1-4}$-Alkyl), $NHC(O)(C_{1-4}$-Alkyl), NHS $(O)_2(C_{1-4}$-Alkyl), C (0) ($C_{1-4}$-Alkyl), $CF_3$ oder $OCF_3$) oder Heterocyclyl (selbst gegebenenfalls substituiert durch Halogen, Hydroxy, Nitro, $NH_2$, $NH(C_{1-4}$-Alkyl), $N(C_{1-4}$-Alkyl$)_2$ (wobei diese Alkylgruppen zu einem Ring verbunden sein können, wie nachstehend für $R^6$ und $R^7$ beschrieben), S $(O)_2(C_{1-4}$-Alkyl), $S(O)_2NH_2$, S $(O)_2NH(C_{1-4}$-Alkyl), $S(O)_2N(C_{1-4}$-Alkyl$)_2$ (wobei diese Alkylgruppen zu einem Ring verbunden sein können, wie nachstehend für $R^6$ und $R^7$ beschrieben), Cyano, $C_{1-4}$-Alkyl, $C_{1-4}$-Alkoxy, C $(O)NH_2$, $C(O)NH(C_{1-4}$-Alkyl), $C(O)N(C_{1-4}$-Alkyl$)_2$ (wobei diese Alkylgruppen zu einem Ring verbunden sein können, wie nachstehend für $R^6$ und $R^7$ beschrieben), $CO_2H$, $CO_2(C_{1-4}$-Alkyl), $NHC(O)(C_{1-4}$-Alkyl), NHS $(O)_2$ ($C_{1-4}$-Alkyl), C (0) ($C_{1-4}$-Alkyl), $CF_3$ oder $OCF_3$) stehen;

$NR^6R^7$ oder $NR^8R^9$ alternativ dazu einen 4- bis 7-gliedrigen heterocyclischen Ring, Azetidin, Pyrrolidin, Piperidin, Azepin, Morpholin oder Piperazin, wobei letzteres gegebenenfalls am distalen Stickstoff durch $C_{1-4}$-Alkyl substituiert ist, bilden können;

oder ein N-Oxid davon oder ein pharmazeutisch annehmbares Salz davon oder ein Solvat davon.

**2.** Verbindung der Formel (I) nach Anspruch 1, worin W für 0 steht.

**3.** Verbindung nach Anspruch 1 oder 2, worin n und m beide für 1 stehen.

**4.** Verbindung nach Anspruch 1, 2 oder 3, worin $R^1$ für gegebenenfalls durch Halogen, Cyano, $C_{1-4}$-Alkyl oder $C_{1-4}$-Alkoxy steht.

**5.** Verbindung der Formel (I) nach einem der vorhergehenden Ansprüche, worin Q für Wasserstoff steht.

**6.** Verbindung der Formel (I) nach einem der vorhergehenden Ansprüche, worin A, B, D, E und G für jeweils eine der Gruppen $CCO_2R^5$, $CR^2$, $CR^3$, $CR^4$ und CH oder N stehen; worin $R^2$, $R^3$ und $R^4$ unabhängig voneinander für Wasserstoff, Halogen, Cyano, Nitro, $C_{1-4}$-Alkyl, $C_{1-4}$-Alkoxy, $CF_3$, $OCF_3$, $S(O)_2(C_{1-4}$-Alkyl) oder $S(O)_2NH_2$ stehen und $R^5$ für Wasserstoff oder $C_{1-6}$-Alkyl steht.

**7.** Verfahren zur Herstellung einer Verbindung der Formel (I) nach Anspruch 1, bei dem man:

a. ein Fluorid (II)

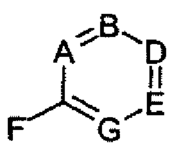

(II)

in Gegenwart eines geeigneten Lösungsmittels bei einer geeigneten Temperatur, gegebenenfalls in Gegenwart einer geeigneten Base mit einer Verbindung der Formel (III):

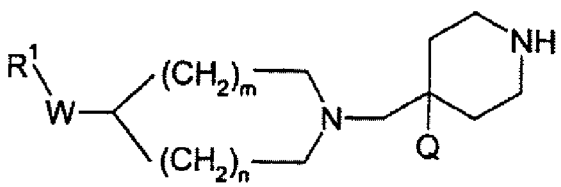

(III)

nucleophil substituiert;

b. wenn $R^5$ für Wasserstoff steht, die Verbindung nach einer in der Technik gut bekannten standardmäßigen Veresterungsmethode in eine erfindungsgemäße Verbindung, in der $R^5$ nicht für Wasserstoff steht, umwandeln kann;

c. wenn $R^5$ nicht für Wasserstoff steht, die Verbindung nach einer in der Technik gut bekannten standardmäßigen Hydrolysemethode in eine erfindungsgemäße Verbindung, in der $R^5$ für Wasserstoff steht, umwandeln kann; oder

d. im Zuge einer Buchwald-Hartwig-Aminierung eine Verbindung der Formel (VIII)

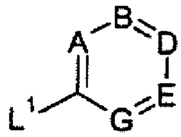

(VIII)

worin $L^1$ für Brom oder Iod steht, in Gegeenwart einer geeigneten Palladiumverbindung, eines Liganden, einer Base und eines Lösungsmittels bei einer geeigneten Temperatur mit einer Verbindung der Formel (III):

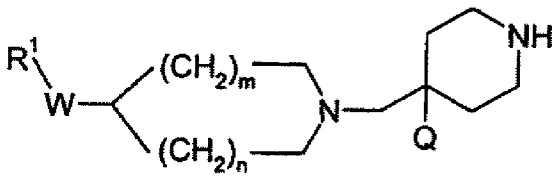

(III)

umsetzt.

**8.** Pharmazeutische Zusammensetzung, die eine Verbindung der Formel (I) oder ein pharmazeutisch annehmbares Salz davon oder Solvat davon nach Anspruch 1 und einen pharmazeutisch annehmbaren Hilfsstoff, ein pharmazeutisch annehmbares Verdünnungsmittel oder einen pharmazeutisch annehmbaren Träger enthält.

**9.** Verbindung der Formel (I) oder ein pharmazeutisch annehmbares Salz davon oder Solvat davon nach Anspruch 1 zur Verwendung bei der Therapie.

**10.** Verbindung der Formel (I) oder ein pharmazeutisch annehmbares Salz davon oder Solvat davon nach Anspruch 1 bei der Herstellung eines Arzneimittels zur Verwendung bei der Therapie.

## Revendications

**1.** Composé de formule (I):

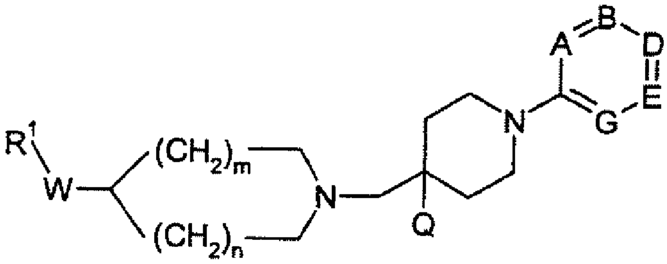

(I)

dans laquelle :

n et m sont indépendamment 0 ou 1 ;
A, B, D, E, G représentent chacun l'un de $CCO_2R^5$, $CR^2$, $CR^3$, $CR^4$, et CH ou N ;
Q est hydrogène ou hydroxy ;
W est $CH_2$, O, NH ou N($C_{1-4}$ alkyle) ;
$R^1$ est phényle éventuellement substitué par halogène, cyano, $C_{1-4}$ alkyle, $C_{1-4}$ halogénoalkyle, $C_{1-4}$ alcoxy ou $C_{1-4}$ halogénoalcoxy ;
$R^2$, $R^3$ et $R^4$ sont indépendamment hydrogène, halogène, cyano, nitro, hydroxy, $NR^6R^7$, $C_{1-6}$ alkyle (éventuellement substitué par halogène), $C_{1-6}$ alcoxy (éventuellement substitué par halogène), $S(O)_p(C_{1-6}$ alkyle), $S(O)_qCF_3$ ou $S(O)_2NR^7R^8$ ;
$R^5$ est hydrogène, $C_{1-6}$ alkyle ou benzyle ;
p et q sont indépendamment 0, 1 ou 2 ;
$R^6$, $R^7$, $R^8$ et $R^9$ sont indépendamment hydrogène, $C_{1-6}$ alkyle (éventuellement substitué par halogène, hydroxy ou $C_{3-6}$ cycloalkyle), $CH_2(C_{2-5}$ alcényle), phényle (lui-même éventuellement substitué par halogène, hydroxy, nitro, $NH_2$, NH($C_{1-4}$ alkyle), N($C_{1-4}$ alkyle)$_2$ (et ces groupements alkyle peuvent se relier pour former un cycle tel que décrit pour $R^6$ et $R^7$ ci-dessous), $S(O)_2(C_{1-4}$ alkyle), $S(O)_2NH_2$, $S(O)_2NH(C_{1-4}$ alkyle), $S(O)_2N(C_{1-4}$ alkyle)$_2$ (et ces groupements alkyle peuvent se relier pour former un cycle tel que décrit pour $R^6$ et $R^7$ ci-dessous), cyano, $C_{1-4}$ alkyle, $C_{1-4}$ alcoxy, $C(O)NH_2$, C(O)NH($C_{1-4}$ alkyle), C(O)N($C_{1-4}$ alkyle)$_2$ (et ces groupements alkyle peuvent se relier pour former un cycle tel que décrit pour $R^6$ et $R^7$ ci-dessous), $CO_2H$, $CO_2(C_{1-4}$ alkyle), NHC(O)($C_{1-4}$ alkyle), $NHS(O)_2(C_{1-4}$ alkyle), C(O)($C_{1-4}$ alkyle), $CF_3$ ou $OCF_3$) ou hétérocyclyle (lui-même éventuellement substitué par halogène, hydroxy, nitro, $NH_2$, NH($C_{1-4}$ alkyle), N($C_{1-4}$ alkyle)$_2$ (et ces groupements alkyle peuvent se relier pour former un cycle tel que décrit pour $R^6$ et $R^7$ ci-dessous), $S(O)_2(C_{1-4}$ alkyle), $S(O)_2NH_2$, $S(O)_2NH(C_{1-4}$ alkyle), $S(O)_2N(C_{1-4}$ alkyle)$_2$ (et ces groupements alkyle peuvent se relier pour former un cycle tel que décrit pour $R^6$ et $R^7$ ci-dessous), cyano, $C_{1-4}$ alkyle, $C_{1-4}$ alcoxy, $C(O)NH_2$, C(O)NH($C_{1-4}$ alkyle), C(O)N($C_{1-4}$ alkyle)$_2$ (et ces groupements alkyle peuvent se relier pour former un cycle tel que décrit pour $R^6$ et $R^7$ ci-dessous), $CO_2H$, $CO_2(C_{1-4}$ alkyle), NHC(O)($C_{1-4}$ alkyle), $NHS(O)_2(C_{1-4}$ alkyle), C(O)($C_{1-4}$ alkyle), $CF_3$ ou $OCF_3$) ;
de manière alternative $NR^6R^7$ ou $NR^8R^9$ peuvent former indépendamment un cycle hétérocyclique à 4 à 7 chaînons, l'azétidine, la pyrrolidine, la pipéridine, l'azépine, la morpholine ou la pipérazine, cette dernière étant éventuellement substituée par $C_{1-4}$ alkyle sur l'azote distal ;
ou un N-oxyde de celui-ci, ou un sel pharmaceutiquement acceptable de celui-ci, ou un solvate de celui-ci.

**2.** Composé de formule (I) selon la revendication 1, **caractérisé en ce que** W est O.

**3.** Composé selon la revendication 1 ou 2, **caractérisé en ce que** n et m sont tous deux 1.

**4.** Composé selon la revendication 1, 2 ou 3, **caractérisé en ce que** $R^1$ est phényle éventuellement substitué par halogène, cyano, $C_{1-4}$ alkyle ou $C_{1-4}$ alcoxy.

**5.** Composé de formule (I) selon l'une quelconque des revendications précédentes, **caractérisé en ce que** Q est hydrogène.

**6.** Composé de formule (I) selon l'une quelconque des revendications précédentes, **caractérisé en ce que** A, B, D, E, G représente chacun l'un de $CCO_2R^5$, $CR^2$, $CR^3$, $CR^4$, et CH ou N ; où $R^2$, $R^3$ et $R^4$ sont indépendamment hydrogène, halogène, cyano, nitro, $C_{1-4}$ alkyle, $C_{1-4}$ alcoxy, $CF_3$, $OCF_3$, $S(O)_2(C_{1-4}$ alkyle) ou $S(O)_2NH_2$ et $R^5$ est hydrogène ou $C_{1-6}$ alkyle.

**7.** Procédé de préparation d'un composé de formule (I) selon la revendication 1, le procédé comprenant :

a. la substitution nucléophile de fluorure (II) :

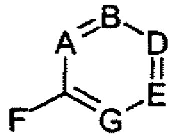

(II)

avec un composé de formule (III) :

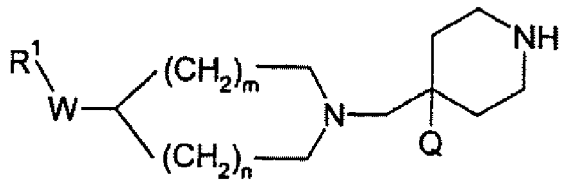

(III)

en présence d'un solvant convenable, à une température convenable, éventuellement en présence d'une base convenable ;

b. lorsque $R^5$ est hydrogène, ledit composé peut être transformé en un composé de l'invention, où $R^5$ n'est pas hydrogène par une méthode d'estérification standard bien connue dans l'art ;

c. lorsque $R^5$ n'est pas hydrogène, ledit composé peut être transformé en un composé de l'invention où $R^5$ est hydrogène par une méthode d'hydrolyse d'ester standard bien connue dans l'art ; ou

d. une amination de Buchwald-Hartwig, où un composé de formule (VIII) :

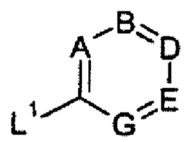

(VIII)

dans laquelle $L^1$ est bromo ou iodo ; est mis en réaction avec un composé de formule (III) :

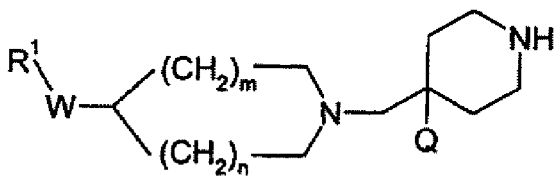

(III)

en présence d'un composé de palladium convenable, d'un ligand, d'une base et d'un solvant, à une température convenable.

**8.** Composition pharmaceutique, **caractérisée en ce qu'**elle comprend un composé de formule (I) ou un sel pharmaceutiquement acceptable de celui-ci ou un solvate de celui-ci selon la revendication 1, et un adjuvant, un diluant ou un support pharmaceutiquement acceptable.

**9.** Composé de formule (I) ou un sel pharmaceutiquement acceptable de celui-ci ou un solvate de celui-ci selon la revendication 1, destiné à être utilisé en thérapie.

**10.** Composé de formule (I) ou un sel pharmaceutiquement acceptable de celui-ci ou un solvate de celui-ci selon la revendication 1, dans la fabrication d'un médicament destiné à être utilisé en thérapie.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9938514 A
- WO 9904794 A
- WO 0035877 A
- WO 0177101 A
- WO 02081449 A
- WO 02079194 A
- WO 0066559 A
- WO 9722596 A
- WO 9730035 A
- WO 9732856 A
- WO 9813354 A
- WO 9902166 A
- WO 0040529 A
- WO 0041669 A
- WO 0192224 A
- WO 0204434 A
- WO 0208213 A


### Non-patent literature cited in the description


- **GREIFF L et al.** *Allergy,* 1999, vol. 54 (11), 1204-8
- **KAWAGUCHI M et al.** *Int. Arch. Allergy Immunol.,* 2000, vol. 122, S1 44
- Protective Groups in Organic Synthesis. 1999
- **HANSEL et al.** *J. Immunol. Methods,* 1991, vol. 145, 105-110
- **STRATH et al.** *J. Immunol. Methods,* 1985, vol. 83, 209
- **HARRISON, R.W.S. ; CARSWELL, H. ; YOUNG, J.M.** *European J. Pharmacol.,* 1984, vol. 106, 405-409